(19) Europäisches Patentamt
European Patent Office
Office européen des brevets

(11) Veröffentlichungsnummer: **0 203 308**
**A2**

(12) **EUROPÄISCHE PATENTANMELDUNG**

(21) Anmeldenummer: **86104231.5**

(22) Anmeldetag: **27.03.86**

(51) Int. Cl.⁴: **C07C 121/66**, C07C 121/75, C07D 211/76, A61K 31/275, A61K 31/445

Die Bezeichnung der Erfindung wurde geändert (Richtlinien für die Prüfung im EPA, A-III, 7.3).

(30) Priorität: **04.04.85 FR 8505123**

(43) Veröffentlichungstag der Anmeldung: **03.12.86 Patentblatt 86/49**

(84) Benannte Vertragsstaaten: **AT BE CH DE FR GB IT LI LU NL SE**

(71) Anmelder: **LABORATOIRES HOECHST S.A.**
**Tour Roussel Nobel 3, Avenue du Général de Gaulle**
**F-92800 Puteaux(FR)**

(72) Erfinder: **Dreux, Jacques, Prof.**
**36, Rue Raulin**
**F-69007 Lyon(FR)**
Erfinder: **Petit, Serge**
**113, Rue Francis de Pressensé**
**F-69100 Villeurbanne(FR)**

(74) Vertreter: **Meyer-Dulheuer, Karl-Hermann, Dr. et al**
**HOECHST Aktiengesellschaft Zentrale Patentabteilung Postfach 80 03 20**
**D-6230 Frankfurt/Main 80(DE)**

(54) **2,3- Oder 3,4-Diphenyl-gamma-nitril-esterderivate zur Behandlung von Niereninsuffizienz und deren Ringschlussprodukte als psychostimulierende und antidepressive Arzneimittel.**

(57) Neue Arzneimittel, bestehend aus 2,3-oder 3,4-Diphenyl-γ-nitril-esterderivaten oder deren Ringschlussprodukten, neue 2,3-und 3,4-Diphenyl-γ-nitril-esterderivate und deren Ringschlussprodukte sowie Verfahren zu deren Herstellung.

Neue Arzneimittel, bestehend aus 2,3-oder 3,4-Diphenyl-γ-nitril-esterderivaten oder den Ringschlussprodukten der 2,3-Diphenylderivate gemäss den beanspruchten Formeln A, B und C.

Ringschlussverfahren für 2,3-Diphenyl-γ-nitril-esterderivate, neue 2,3-Diphenyl-γ-nitril-esterderivate der beanspruchten Formeln I bis VIII; neue 3,4-Diphenylderivate der besagten Ester, entsprechend den Formeln IX und X, sowie neue 3,4-Diphenyl-piperidinonderivate der spezifischen Formeln XI bis XXVII.

EP 0 203 308 A2

Neue Arzneimittel, bestehend aus 2,3-oder 3,4-Diphenyl-γ-nitril-esterderivaten oder deren Ringschlussprodukten, neue 2,3-bzw. 3,4-Diphenyl-γ-nitril-esterderivate und Ringschlussprodukte sowie Verfahren zu deren Herstellung.

Vorliegende Erfindung betrifft neue Arzneimittel, die aus 2,3-oder 3,4-Diphenyl-γ-nitril-esterderivaten oder deren Ringschlussprodukten bestehen, neue 2,3-oder 3,4-Diphenyl-γ-nitril-esterderivate und deren Ringschlussprodukte sowie Verfahren zu deren Herstellung und ihre Verwendung als Arzneimittel, vorzugsweise zur Behandlung akuter und chronischer Niereninsuffizienz (Nitrilester) sowie als Psychostimulanz und Antidepressivum.

In der Literatur finden sich einige Angaben über die Reihen der 2,3-oder 3,4-Diphenyl-γ-nitrilester (siehe insbesondere die Arbeiten von:

-C.F. KOELSCH, J.A.C.S., 65, 437 -9 (1943)

-POPANDOVA, K., IVANOV, K.H., God. Sofii. Univ. Khim. Fak. 1970 -1971 (veröffentlicht 1973), 65 252-60 (bulgarisch),

was die 2,3-Diphenylderivate angeht, und die Arbeiten von

-S. AVERY, J.A.C.S., 50, 2512 -9 (1928),

-C.F. KOELSCH, J.A.C.S., 65, 437 -9 (1943)

-W.M. BARR und J.W. COOK, J. Chem. Soc., 438 -41 (1945)

-R. BERTOCCHIO und J. DREUX, Bull. Soc. Chim. Fr., 1809 -13 (1962),

mit bezug auf die 3,4-Diphenyl-γ-nitril-esterderivate).

Es finden sich erheblich weniger Literaturstellen für die Piperidinone, d.h. die Ringschlussprodukte der obigen γ-Nitril-ester (zum Beispiel MORTIMER in Aust. J. Chem. 21, 467-76, 1968).

Die Anmelderin hat nun gefunden, dass diese sowie zahlreiche weitere neue, nicht beschriebene, von ihr synthetisierte und zur gleichen Gruppe gehörende Produkte bemerkenswerte therapeutische Eigenschaften besitzen.

Gegenstand der vorliegenden Erfindung sind somit neue Arzneimittel, die dadurch gekennzeichnet sind, dass sie aus 2,3-oder 3,4-Diphenyl-γ-nitril-esterderivaten oder den Ringschlussprodukten der 2,3-Diphenylderivate der nachfolgenden Formeln A, B und C bestehen oder diese Verbindungen enthalten

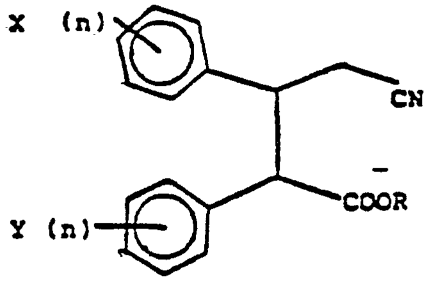

(A)

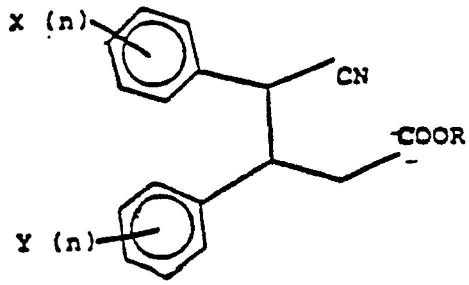

(B)

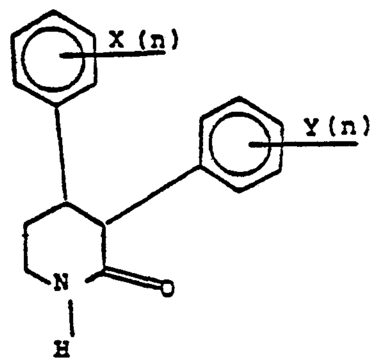

(C)

Formel A stellt die 2,3-Diphenyl-γ-nitril-ester, Formel B die 3,4-Diphenyl-γ-nitril-ester und Formel C die Ringschlussprodukte der 2,3-Diphenyl-γ-nitril-ester dar, wobei in den Formeln R für ein Wasserstoffatom, ein pharmazeutisch unbedenkliches Alkali-oder Erdalkalimetall oder eine $C_1$-$C_4$-Alkylgruppe sowie X und Y, die gleich oder verschieden sein können, für ein Wasserstoff-oder Halogenatom oder eine $C_1$-$C_4$-Alkyl-, $C_1$-$C_4$-Alkoxy-, Phenyl-, Phenyl-$C_1$-$C_4$-alkyl-oder Trifluormethylgruppe in den Formeln A und B bzw. X und Y, die gleich oder verschieden sein können, für ein Wasserstoff-oder Halogenatom oder eine Trifluormethyl-, $C_1$-$C_4$-Alkyl-, Phenyl-, Phenyl-$C_1$-$C_4$-alkyl-, $C_1$-$C_4$-Alkoxy-, Hydroxyl-, Acyloxy-, Phenyloxy-, Benzyloxy-, $C_1$-$C_4$-Alkyl-sulfonyl-oder Di-$C_1$-$C_4$-alkylaminogruppe in der Formel C stehen, wobei n 1 bis 3 beträgt, falls X und/oder Y von Wasserstoff verschieden sind.

Die durch die Formeln A und B dargestellten Produkte können die erythro-oder threo-bzw. im Fall der Piperidinone (Formel C) die cis-oder trans-Konfiguration besitzen.

Die Herstellung gewisser obengenannter Derivate ist zwar in der Literatur beschrieben (was insbesondere für die unsubstituierten Diphenyle der Fall ist, siehe KOELSCH, a.a.O.), aber deren Ringschluss zu den reinen cis-oder trans-Diastereoisomeren ist unbekannt und neu. Es ist in der Tat sehr wichtig, die reinen Diastereoisomeren zur Verfügung zu haben, da die therapeutische Aktivität sowohl qualitativ als auch quantitativ eine Funktion der cis-oder trans-Konfiguration des Moleküls ist.

Gegenstand vorliegender Erfindung ist demnach weiterhin das Ringschlussverfahren für 2,3-Diphenyl-γ-nitril-esterderivate unter Festlegung der Stereochemie der entstehenden Piperidinone, welches dadurch gekennzeichnet ist, dass man, wenn ein reines cis-Derivat erwünscht ist, von einem reinen erythro-Produkt ausgeht und dieses bei einem Druck zwischen 10 und 80 bar und einer Temperatur zwischen 30 und 100°C hydriert bzw., wenn ein reines trans-Derivat erwünscht ist, entweder von einem reinen threo-Derivat oder einem erythro-Derivat ausgeht, das bei einem Druck zwischen 80 und 180 bar und einer Temperatur zwischen 80 und 180°C hydriert wird.

Die Anmelderin hat zeigen können, dass diese ganze erfindungsgemässe Gruppe verschiedene therapeutische Wirkungen besitzt, wie beispielsweise eine gefässerweiternde Wirkung für die γ-Nitril-ester bzw. einen psychostimulierenden und antidepressiven Effekt im Fall der Piperidinone.

Vorliegende Erfindung betrifft somit neue erfindungsgemässe Arzneimittel sowie die zu deren Herstellung und Verwendung angewandten Methoden.

Dem besseren Verständnis der Erfindung dient die folgende ergänzende Beschreibung, die sich auf Herstellungsbeispiele für die erfindungsgemässen Produkte sowie einen Bericht der Untersuchungen über die pharmakologische Aktivität der erfindungsgemässen Produkte bezieht.

Die Produkte der Formeln A und B werden nach in der Literatur beschriebenen Methoden hergestellt.

Es versteht sich jedoch, dass diese Beispiele und Berichte ausschliesslich zur Erläuterung des Erfindungsgegenstands gegeben werden und in keiner Weise eine Einschränkung darstellen.

A -Herstellungsbeispiele für γ-Nitril-ester des 2,3-Diphenyltyps und Derivate.

Beispiel 1: Herstellung von erythro-4-Cyan-3-(4-methoxyphenyl)-2-phenylbuttersäureäthylester 1

Man gibt 20 g (0,125 Mol) para-Methoxyzimtsäurenitril in 40 $cm^3$ wasserfreiem Aether gelöst unter Rühren und unter Stickstoff zusammen mit 5,8 g (0,148 Mol) Natriumamid in einen mittels eines Eis/Salzbads auf -10°C gehaltenen 250 $cm^3$-Kolben.

Im Verlauf von 25 Minuten versetzt man mit 21,4 g (0,130 Mol) Phenylessigsäureäthylester gelöst in 40 $cm^3$ wasserfreiem Aether. Innerhalb 1 Stunde und 15 Minuten lässt man die Temperatur bis auf +10°C ansteigen. Das Gemisch verdickt sich dann schnell und erstarrt zu einer Masse, die man über Nacht im Kühlschrank stehen lässt.

Das Gemisch wird bei -5° bis -10°C (Eis/Salz) mit 10 $cm^3$ Wasser und 75 $cm^3$ 4 n-HCl hydrolysiert. Um das Gemisch homogener zu machen und eine pH-Einstellung auf 7 zu gestatten, muss man Methylenchlorid (80 $cm^3$) zusetzen.

Man löst das gebildete Produkt vollständig durch Zusatz von 100 $cm^3$ Methylenchlorid, giesst ab und trennt die organische Phase ab. Die wässrige Phase wird mit Methylenchlorid (2 x 20 $cm^3$) extrahiert. Die organischen Phasen werden vereinigt und über wasserfreiem Natriumsulfat getrocknet.

Nach dem Eindampfen erhält man 39,7 g - (97%) eines festen Rückstands, der ausschliesslich das erythro-Racemat enthält. Nach Umkristallisieren aus absolutem Aethanol (180 $cm^3$) erhält man 35,8 g (88%) weisses Produkt, das dem reinen erythro-Racemat entspricht. Schmelzpunkt = 125,5 -126,5°C.

Analyse: $C_{20}H_{21}NO_3$

| | C | H | N | O |
|---|---|---|---|---|
| Berechnet | 74,46 | 6,57 | 4,41 | 14,75 |
| Gefunden | 74,28 | 6,54 | 4,33 | 14,84 |

IR (KBr): ν C≡N, 2240 $cm^{-1}$, schwach
ν C=O, 1720 $cm^{-1}$, stark

Massenspektrometrie: (70 eV), M/Z (relative Intensität)

323 (12, $M^+$), 210 (10), 167 (5), 165 (9), 164 (13), 161 (22), 160 (100), 152 (5), 145 (9), 118 (5), 117 (4), 105 (6), 91 (5), 89 (4), 77 (5).

NMR 80 MHz:

$C_6D_6$: Signale bei δ :

7,50 -7,00 ppm (7 H, Ar, breiter Komplex)

7,75 ppm ( 2 H, Ar, d., $^J H_{meta}$ -H $_{ortho}$ = 9 Hz)

4,02 ppm (1 H2, d., $J_{H2-H3}$ = 12 Hz)

3,80 -3,20 ppm (6 H, breiter Komplex, dabei Methoxy bei 3,30 ppm)

1,72 ppm (2 $H_4$, $J_{H3-H4}$ = 5 Hz)

0,57 ppm ( 3 H, t.)

$CDCl_3$: Signale bei $\delta$ :

7,60 -7,20 ppm (7 H, Ar, breiter Komplex)

6,87 ppm (2 H, Ar, D., $^{J}H_{ortho}$ -$H_{meta}$ = 10 Hz)

4,10 -3,30 ppm (7 H, breiter Komplex, dabei Methoxy bei 3,80 ppm)

2,30 ppm (2 $H_4$, m.)

0,92 ppm (3 H, t.)

Beispiel 2: Herstellung von erythro-4-Cyan-2-(4-chlorphenyl)-3-phenylbuttersäureäthylester 2

Man gibt 6,5 g (0,050 Mol) Zimtsäurenitril in 10 $cm^3$ wasser freiem Aether gelöst unter Rühren und unter Stickstoff zusammen mit 2,55 g (0,065 Mol) Natriumamid in einen mittels eines Eis/Salzbads auf -5°C gehaltenen 100 $cm^3$-Reaktor.

Im Verlauf von 15 Minuten versetzt man mit 12 g (0,060 Mol) para-Chlorphenylessigsäureäthylester gelöst in 10 $cm^3$ wasserfreiem Aether.

Man lässt die Reaktion 45 Minuten lang bei -5°C und dann 30 Minuten lang bei +5°C ablaufen.

Nach Behandlung gemäss Beispiel 1 erhält man 2 in 69% Ausbeute. Schmelzpunkt = 97 - 98°C.

Analyse: $C_{19}H_{18}ClNO_2$

| | C | H | Cl | N |
|---|---|---|---|---|
| Berechnet | 69,62 | 5,52 | 10,81 | 4,27 |
| Gefunden | 69,47 | 5,45 | 11,07 | 4,29 |
| | | | 11,19 | |

Beispiel 3: Herstellung von threo-4-Cyan-2-(4-chlorphenyl)-3-phenylbuttersäureäthylester 3

Man löst 10 g (0,030 Mol) 2 in 100 $cm^3$ äthanolischer 0,1 n-Kalilauge. Nach 5 Minuten Erhitzen zum Rückfluss neutralisiert man bei +15°C mit 1 n-HCl. Der nach Eindampfen erhaltene Rückstand wird in 100 $cm^3$ Dichlormethan gelöst; die organische Phase wird mit Wasser (2 x 20 $cm^3$) gewaschen und dann über wasserfreiem Natriumsulfat getrocknet. Dabei erhält man in quantitativer Ausbeute ein 35% 3 und 65% 2 enthaltendes Gemisch.

Das Produkt 3 wird durch "flash"-Chromatographie isoliert:

Kieselgel 60 -230-400 Mesh, h = 25 cm, $^{P}N_2$ = 0,2 bar,

Laufmittel: Hexan/Essigester 90/10. Schmelzpunkt = 104°C

(absolutes Aethanol). Ausbeute: 30%.

Beispiel 4: Herstellung von erythro-4-Cyan-3-(4-chlorphenyl)-2-phenylbuttersäureäthylester 4

Man gibt 39 g (0,238 Mol) geschmolzenes und dann in 60 $cm^3$ wasserfreiem Aether gelöstes 4-Chlor-zimtsäurenitril unter Rühren und unter Stickstoff zusammen mit 11 g (0,282 Mol) Natriumamid in einen mittels eines Eis/Salzbads auf -5°C gehaltenen 500 $cm^3$-Kolben.

Im Verlauf von 35 Minuten versetzt man mit 43 g (0,261 Mol) Phenylessigsäureäthylester gelöst in 100 $cm^3$ wasserfreiem Aether.

Man lässt 45 Minuten lang bei Raumtemperatur stehen, bevor man das Reaktionsgemisch gemäss Beispiel 1 behandelt -Ausbeute: 80%, Schmelzpunkt = 124°C.

<u>Analyse:</u> $C_{19}H_{18}ClNO_2$

| | C | H | N | Cl |
|---|---|---|---|---|
| Berechnet | 69,62 | 5,52 | 4,27 | 10,81 |
| Gefunden | 69,61 | 5,49 | 4,27 | 11,08 |
| | | | | 11,18 |

<u>Beispiel</u> <u>5:</u> Herstellung von erythro-4-Cyan-3-(3,4-dichlorphenyl)-2-phenylbuttersäureäthylester <u>5</u>

Man gibt 11,2 g (0,056 Mol) 3,4-Dichlor-zimtsäurenitril in einem Gemisch aus Methylenchlorid (15 $cm^3$) und wasserfreiem Aether (50 $cm^3$) gelöst unter Rühren und unter Stickstoff zusammen mit 2,7 g (0,069 Mol) Natriumamid in einen mittels eines Eis/Salzbads auf -8°C gehaltenen 250 $cm^3$-Kolben.

Im Verlauf von 15 Minuten versetzt man mit 10,3 g (0,062 Mol) Phenylessigsäureäthylester gelöst in 10 $cm^3$ wasserfreiem Aether.

Man lässt die Reaktion 1 Stunde lang bei -8°C ablaufen, bevor man die Temperatur auf +5°C ansteigen lässt.

Nach Behandlung gemäss Beispiel 1 erhält man <u>5</u> in 65% Ausbeute. Schmelzpunkt = 117,5 - 118°C.

<u>Analyse:</u> $C_{19}H_{17}Cl_2NO_2$

| | C | H | O | N | Cl |
|---|---|---|---|---|---|
| Berechnet | 62,99 | 4,72 | 8,83 | 3,86 | 19,57 |
| Gefunden | 62,78 | 4,79 | 8,55 | 3,85 | 19,35 |

<u>Beispiel</u> <u>6:</u> Herstellung von erythro-4-Cyan-3-(4-methylphenyl)-2-phenylbuttersäureäthylester <u>6</u>

Man gibt 22 g (0,153 Mol) geschmolzenes 4-Methyl-zimtsäurenitril, 20 $cm^3$ wasserfreien Aether und 7 g (0,179 Mol) Natriumamid unter Rühren und unter Stickstoff in einen auf 12-13°C gehaltenen 250 $cm^3$-Reaktor.

Im Verlauf von 45 Minuten lässt man 27 g - (0,164 Mol) Phenylessigsäureäthylester einlaufen.

Nach Behandlung gemäss Beispiel 1 erhält man <u>6</u> in 77% Ausbeute -Schmelzpunkt = 120 - 120,5°C (absolutes Aethanol/ Aether -60/40).

<u>Analyse:</u> $C_{20}H_{21}NO_2$

| | C | H | N | O |
|---|---|---|---|---|
| Berechnet | 78,15 | 6,87 | 4,55 | 10,41 |
| Gefunden | 78,17 | 6,95 | 4,76 | 10,37 |

Beispiel 7: Herstellung von erythro-4-Cyan-2-(3,4-dimethoxyphenyl)-3-phenylbuttersäureäthylester 7

Man versetzt ein Gemisch aus 6 g (0,026 Mol) 3,4-Dimethoxyphenylessigsäureäthylester in 30 $cm^3$ wasserfreiem Aether gelöst und 1,1 g (0,028 Mol) Natriumamid unter Rühren und unter Stickstoff bei Rückfluss des Aethers im Verlauf von 20 Minuten mit 3,45 g (0,026 Mol) Zimtsäurenitril, gelöst in 10 $cm^3$ wasserfreiem Aether.

Man hält 7 Stunden lang unter Rückfluss. Bei Behandlung des Reaktionsgemischs gemäss Beispiel 1 erhält man 7 in 69% Ausbeute. Schmelzpunkt = 144°C.

Analyse: $C_{21}H_{23}NO_4$

| | C | H | N | O |
|---|---|---|---|---|
| Berechnet | 71,37 | 6,55 | 3,96 | 18,10 |
| Gefunden | 71,32 | 6,53 | 4,09 | 18,06 |

Beispiel 8: Herstellung von erythro-4-Cyan-3-(4-trifluormethylphenyl)-2-phenylbuttersäureäthylester 8

Man gibt 20 g (0,101 Mol) 4-Trifluormethylzimtsäurenitril in 100 $cm^3$ wasserfreiem Aether gelöst unter Rühren und unter Stickstoff zusammen mit 5 g (0,128 Mol) Natriumamid in einen mittels eines Eis/Salzbads auf -5°C gehaltenen 250 $cm^3$ Kolben.

Im Verlauf von 40 Minuten versetzt man mit 18,4 g (0,112 Mol) Phenylessigsäureäthylester, gelöst in 30 $cm^3$ wasserfreiem Aether. Man lässt die Temperatur bis auf +5°C steigen und rührt 3 Stunden lang bei dieser Temperatur. Behandlung des Reaktionsgemischs gemäss Beispiel 1 ergibt 8 in 69% Ausbeute. Schmelzpunkt = 141,5 - 142,5°C.

Analyse: $C_{20}H_{18}F_3NO_2$

| | C | H | F | N | O |
|---|---|---|---|---|---|
| Berechnet | 66,48 | 5,01 | 15,77 | 3,87 | 8,85 |
| Gefunden | 66,21 | 5,07 | 15,70 | 3,87 | 9,15 (als Differenz) |
| | | 4,97 | 15,76 | 3,80 | |

Beispiel 9: Herstellung von 4-Cyan-2-(4-chlorphenyl)-3-phenylbuttersäure 9

Man versetzt 10 g (0,030 Mol) 2 oder 3 gelöst in 100 $cm^3$ Pyridin bei Raumtemperatur mit 40 $cm^3$ (0,040 Mol) wässriger 1n-Natronlauge.

Nach 24 Stunden Rühren giesst man das viskose, teilweise kristallisierte Reaktionsgemisch auf Eis, säuert mit konzentrierter HCl an und extrahiert dann mit Aether. Die vereinigten Aetherphasen werden mit Wasser neutral gewaschen.

Man erhält die Nitrilsäure quantitativ in Form eines Gemischs der Racemate im Verhältnis 62/38.

Umkristallisieren aus Benzol/Heptangemisch 50/50

Ausbeute = 77%

Schmelzpunkt = 155 -165°C

Entsprechende Natriumsalze:

Behandlung des Gemischs der obigen $\gamma$-Nitrilsäuren mit Natriumhydroxyd führt zu einem Gemisch der Natriumsalze. Schmelzpunkt = 260 - 270°C.

B -Herstellungsbeispiele für $\gamma$-Nitril-ester des 3,4-Diphenyltyps

Beispiel 10: Herstellung von erythro-4-Cyan-(4-(3,4-dimethoxyphenyl)-3-phenylbuttersäureäthylester 10

Man gibt 17,62 g (0,100 Mol) Zimtsäureäthylester und 17,72 g (0,100 Mol) 3,4-Dimethoxyphenylacetonitril unter Rühren in einen mit einem Thermostatbad auf 60°C gehaltenen 250 $cm^3$ Kolben.

Im Verlauf von 10 Minuten gibt man 2,5 g -(0,036 Mol) Natriumäthylat gelöst in 10 $cm^3$ absolutem Aethanol dazu und rührt noch 1 Stunde bei 60°C.

Das Gemisch wird bei -5°C mit Wasser und danach verdünnter HCl hydrolysiert. Um das Gemisch homogener zu machen und eine pH-Einstellung auf 7 zu gestatten, muss man Methylenchlorid einsetzen.

Die organische Phase wird mit Wasser gewaschen und dann über wasserfreiem Natriumsulfat getrocknet.

Dabei erhält man 35 g (99%) viskoses Oel, welches das Gemisch der erythro-und threo-Racemate im Verhältnis 75/25 enthält. Das als Hauptbestandteil vorliegende erythro-Isomer wird durch "flash"-Chromatographie unter Verwendung von Hexan/Essigester 75/25 als Eluiermittel isoliert -(Kieselgel 60, Siebgrösse 230-400).

Man gewinnt 21,2 g (60%) farbloses Oel, das in wasserfreiem Aether kristallisiert und reinem erythro-Racemat von Schmelzpunkt 69-70°C entspricht.

Analyse: $C_{21}H_{23}NO_4$

| | C | H | N | O |
|---|---|---|---|---|
| Berechnet | 71,36 | 6,55 | 3,96 | 18,11 |
| Gefunden | 71,26 | 6,48 | 4,09 | 18,20 |

Massenspektrometrie:(70 eV); M/Z (relative Intensität).

353 (41, $M^+$), 308 (11), 265 (8), 178 (16), 177 -(100), 176 (43), 162 (8), 136 (8), 135 (76), 131 (16), 107 (11), 105 (27).

NMR 80 MHz:

$CDCl_3$, Signale bei $\delta$ :

7,40 -7,00 ppm (5 H, Ar, breiter Komplex)

4,27 ppm (1 H4, d., $J_{H3-H4}$ = 6,3 Hz)

4,10 ppm (2 H, q)

3,85 ppm (3 H, s., Methoxy)

3,70 ppm (3 H, s., Methoxy)

3,60 -3,40 ppm (1 H3, breiter Komplex)

3,20 -2,55 ppm (2 H2, breiter Komplex)

1,17 ppm (3 H, t.)

Beispiel 11: Herstellung von threo-4-Cyan-3-(4-chlorophenyl)-4-phenylbuttersäureäthylester 11

Man versetzt 11,7 g (0,100 Mol) Benzylcyanid und 21 g (0,100 Mol) para-Chlorzimtsäureäthylester, bei 0°C vermischt, im Verlauf 1/2 Stunde mit 200 $cm^3$ wasserfreiem Aether, der eine äthanolische Natriumäthylatlösung (entsprechend 2,3 g Natrium) enthält.

Man hält das Raktionsgemisch 5 Stunden lang bei 0°C und hydrolysiert dann mit verdünnter HCl, giesst ab, extrahiert die wässrige Phase mit Aether, vereinigt die organischen Phasen und wäscht sie neutral.

Dabei erhält man das Gemisch der beiden Racemate in 85% Ausbeute.

Das reine threo-Racemat wird durch zweimaliges Umkristallisieren aus absolutem Aethanol isoliert. Schmelzpunkt = 108 -109°C.

Analyse: $C_{19}H_{18}ClO_2N$

| | C | H | Cl |
|---|---|---|---|
| Berechnet | 69,61 | 5,53 | 10,88 |
| Gefunden | 69,40 | 5,42 | 11,06 |

C -Herstellungsbeispiele für Ringschlussprodukte: 3,4-Diphenyl-piperidinone.

Beispiel 12: Herstellung von cis-4-(4-Methoxyphenyl)-3-phenyl-2-piperidinon 12

Man gibt 5 g (0,015 Mol) 4-Cyan-3-(4-methoxyphenyl)-2-phenylbuttersäureäthylester 1 und 1 g 5%ige Platinkohle mit 80 cm³ Eisessig in einen Autoklaven. Unter 80 bar Wasserstoffdruck erhitzt man 1 Stunde und 30 Minuten lang auf 100°C. Nach Abfiltrieren des Katalysators, Abdampfen der Essigsäure und Aufnehmen des entstandenen viskosen Rückstands in 130 cm³ Methylenchlorid neutralisiert man die organische Phase mit gesättigter Natriumbicarbonatlösung, wäscht mit destilliertem Wasser und trocknet über wasserfreiem Natriumsulfat.

Das nach Eindampfen erhaltene Produkt wird in Xylol (70 cm³) gelöst und 4 Stunden lang am Rückfluss erhitzt. Das gebildete Piperidon fällt in der Kälte aus diesem Lösungsmittel aus. Man filtriert und wäscht mit wasserfreiem Aether.

Dabei erhält man 2,75 g (63%) weisses kristallines Produkt, das dem cis-Racemat 12 entspricht. Schmelzpunkt = 187 -188°C.

Analyse: $C_{18}H_{19}NO_2$

| | C | H | N | O |
|---|---|---|---|---|
| Berechnet | 76,84 | 6,80 | 4,97 | 11,37 |
| Gefunden | 76,89 | 6,84 | 4,93 | 11,34 |
| | | 6,83 | | |

IR: (KBr) ν C=O, 1660 $cm^{-1}$, stark
ν N-H, 3180 $cm^{-1}$, mittel

Massenspektrometrie: (70 eV); M/Z (relative Intensität)

281 (71, $M^+$), 147 (100), 134 (59), 118 (73), 91 - (16), 90 (19).

NMR 80 MHz:

$C_6D_6$: Signale bei δ :

8,35 ppm (1 H, s., N-H)

7,35-6,80 ppm (5 H, Ar, breiter Komplex)

6,75-6,35 ppm (4 H, Ar, breiter Komplex)

3,95 ppm (H3, d., $J_{H3-H4}$ = 4,8 Hz)

3,25 ppm (3 H, Singlett, Methoxy)

3,15-2,70 ppm (3 H, breiter Komplex)

2,20-1,60 ppm (2 H, grosser breiter Komplex)

Beispiel 13: Herstellung von trans-4-(4-Methoxyphenyl)-3-phenyl-2-piperidinon 13

Man gibt 5 g (0,015 Mol) erythro-4-Cyan-3-(4-methoxyphenyl)-2-phenylbuttersäureäthylester 1 und 1 g 5%ige Platinkohle mit 50 cm³ Eisessig in eine Hydrierbombe. Man erhitzt unter Stickstoff (-schwacher Ueberdruck) auf 100°C und leitet dann unter hohem Druck (150 bar) Wasserstoff ein und stabilisiert die Temperatur bei 120°C. Nach 1 Stunde Reaktionszeit kühlt man ab, filtriert den Katalysator ab und verdampft die Essigsäure.

Das dabei entstandene Oel wird in Xylol aufgenommen und 4 Stunden lang am Rückfluss erhitzt. Man verdampft das Xylol und nimmt in Methylenchlorid auf, um durch Waschen mit gesättigter Natriumbicarbonatlösung und dann mit destilliertem Wasser zu neutralisieren.

Nach Trocknen der organischen Phase über wasserfreiem Natriumsulfat, Filtrieren und Abdampfen des Lösungsmittels erhält man 4,35 g (100%) viskosen Rückstand, der durch Zusatz von wasserfreiem Aether ausgefällt wird.

Nach Umkristallisieren aus absolutem Aethanol/Aether (70/30) erhält man 3,2 g (73%) kristallisiertes Produkt vom Schmelzpunkt 144-146°C, das dem reinen trans-Racemat 13 entspricht.

Analyse: $C_{18}H_{19}NO_2$

| | C | H | N | O |
|---|---|---|---|---|
| Berechnet | 76,84 | 6,80 | 4,97 | 11,37 |
| Gefunden | 76,64 | 6,91 | 4,46 | 11,50 |
| | 76,78 | 7,16 | 4,61 | 11,61 |

IR: (KBr) ν C=O, 1655 $cm^{-1}$, stark
ν N-H, 3170 $cm^{-1}$, mittel

Massenspektrometrie: (70 eV); M/Z (relative Intensität)

281 (60; $M^+$), 147 (100,) 134 (66), 118 (71), 91 - (34).

NMR 80 MHz:

$C_6D_6$: Signale bei δ :

8,45 ppm (1 H, s, N-H)

7,05 ppm (5 H, Ar, breiter Komplex)

6,65 ppm (4 H, Ar, breiter Komplex)

3,60 ppm (1 H3, d, $J_{H3-H4}$ = 10 Hz)

3,20 ppm (3 H, s, Methoxy)

3,05-2,65 ppm (3 H, breiter Komplex)

1,70-1,35 ppm (2 H, breiter Komplex)

Beispiel 14: Herstellung von cis-3-(4-Chlorphenyl)-4-Phenyl-2-piperidinon 14

Man gibt 13, 5 g (0,041 Mol) 2 und 2 g 5%ige Platinkohle mit 70 cm³ Eisessig in einen Autoklaven. Man erhitzt unter einem Druck von 10 bar 4 Stunden lang auf °C.

Nach Behandlung gemäss Beispiel 12 erhält man 14 in 51% Ausbeute -Schmelzpunkt = 180 - 181°C (Xylol).

Analyse: $C_{17}H_{16}ClNO$

| | C | H | Cl | N |
|---|---|---|---|---|
| Berechnet | 71,45 | 5,63 | 12,40 | 4,90 |
| Gefunden | 71,42 | 5,57 | 12,64 | 4,70 |

Beispiel 15: Herstellung von trans-3-(4-Chlorphenyl)-4-phenyl-2-piperidinon 15

1. Weg: Man gibt 6,6 g (0,020 Mol) 2 und 0,8 g 5%ige Platinkohle mit 65 $cm^3$ Eisessig in einem Autoklaven. Unter schwachem Stickstoffdruck erhitzt man auf 90°C und leitet Wasserstoff unter hohem Druck (130 bar) erst ein, nachdem sich die Temperatur auf 90°C stabilisiert hat. Nach 1 Stunde Reaktionszeit behandelt man das Gemisch gemäss Beispiel 12.

Das dabei erhaltene Rohprodukt setzt sich jedoch aus 90% des trans-Racemats 15 und 10% des cis-Racemats 14 zusammen.

15 wird durch Hochdruckflüssigkeitschromatographie auf einer semipräparativen Partisil-10-Säule unter Verwendung von Essigester/Methanol 97/3 als Eluiermittel abgetrennt. -Ausbeute: 72%. Schmelzpunkt = 139 - 141°C.

Analyse: $C_{17}H_{16}ClNO$

| | C | H | Cl | N |
|---|---|---|---|---|
| Berechnet | 71,45 | 5,63 | 12,40 | 4,90 |
| Gefunden | 71,44 | 5,34 | 12,51 | 4,93 |
| | | | 12,48 | |

2. Weg: Man behandelt 2,6 g (7,9 . $10^{-3}$ Mol) 3 und 0,3 g 5%ige Platinkohle gemäss Beispiel 14, wobei man 15 in 65% Ausbeute erhält.

Beispiel 16: Herstellung von cis-4-(4-Chlorphenyl)-3-phenyl-2-piperidinon 16

Man gibt 10 g (0,030 Mol) 4 und 2 g 5%ige Platinkohle mit 150 $cm^3$ Eisessig in einen Autoklaven. Man erhitzt 5 Stunden lang unter 40 bar Druck auf 80°C.

Nach Filtrieren, Neutralisieren und Umkristallisieren aus einem ternären Gemisch von wasserfreiem Aether, Hexan und Methylenchlorid (60/20/20) erhält man 16 in 77% Ausbeute mit Schmelzpunkt = 168°C.

Analyse: $C_{17}H_{16}ClNO$

| | C | H | N | O | Cl |
|---|---|---|---|---|---|
| Berechnet | 71,45 | 5,63 | 4,90 | 5,59 | 12,40 |
| Gefunden | 71,25 | 5,57 | 4,86 | 5,70 | 11,83 |
| | 71,47 | 5,58 | 5,00 | 5,54 | 12,07 |

Beispiel 17: Herstellung von trans-4-(4-Chlorphenyl)-3-phenyl-2-piperidinon 17

Man gibt 10 g (0,030 Mol) 4 und 2 g 5%ige Platinkohle mit 80 cm³ Eisessig in einen Autoklaven. Man erhitzt unter geringem Stickstoffdruck auf 180°C und leitet Wasserstoff unter hohem Druck - (180 Atmosphären) erst nach Stabilisierung der Temperatur ein. Nach 30 Minuten Rühren erhält man ein Gemisch des trans-Racemats 17 und cis-Racemats 16 im Verhältnis 90/10.

Nach Trennung gemäss Beispiel 15 (1. Weg) und Umkristallisieren aus Aceton erhält man 17 in 50 % Ausbeute. Schmelzpunkt = 156-157°C.

Analyse: $C_{17}H_{16}NClO$

| | C | H | N | Cl |
|---|---|---|---|---|
| Berechnet | 71,45 | 5,63 | 4,90 | 12,40 |
| Gefunden | 71,21 | 5,46 | 4,72 | 12,33 |
| | | | | 12,42 |

Beispiel 18: Herstellung von cis-4-(3,4-Dichlorphenyl)-3-phenyl-2-piperidinon 18

Behandlung von 5 unter den Bedingungen des Beispiels 16 und nachfolgendes 2 Stunden und 30 Minuten langes Erhitzen unter Rückfluss in Xylol und Umkristallisieren aus Aceton führt zu 18 in 50% Ausbeute -Schmelzpunkt = 169°C.

Analyse: $C_{17}H_{15}NOCl_2$

| | C | H | N |
|---|---|---|---|
| Berechnet | 63,76 | 4,72 | 4,37 |
| Gefunden | 62,80 | 4,72 | 4,54 |
| | 63,83 | 4,94 | 4,35 |

Beispiel 19: Herstellung von trans-4-(3,4-dichlorphenyl)-3-phenyl-2-piperidinon 19

Man gibt 10 g (0,027 Mol) 5 und 2 g 5%ige Platinkohle mit 60 cm³ Eisessig in einen Autoklaven. Man erhitzt unter leichtem Stickstoffdruck auf 150°C und leitet Wasserstoff unter hohem Druck - (175 bar) erst nach Stabilisierung der Temperatur ein. Nach 1 Stunde und 15 Minuten Reaktionszeit erhält man eine Gemisch der trans/cis-Racemate im Verhältnis 93/7.

Nach Abtrennung gemäss Beispiel 15 (1. Weg) und Umkristallisieren aus Aceton erhält man 19 in 48% Ausbeute -Schmelzpunkt = 154-155°C.

Analyse: $C_{17}H_{15}NCl_2O$

| | C | H | N | Cl |
|---|---|---|---|---|
| Berechnet | 63,76 | 4,72 | 4,37 | 22,14 |
| Gefunden | 63,54 | 4,67 | 4,30 | 22,04 |
| | 63,61 | 4,71 | 4,31 | |

Beispiel 20: Herstellung von cis-4-(4-Methylphenyl)-3-phenyl-2-piperidinon 20

Man rührt 20 g (0,065 Mol) 6 und 130 $cm^3$ Eisessig 6 Stunden lang bei 90°C unter 40 bar Wasserstoffdruck in Gegenwart von Raney-Nickel. Nach Behandlung in absolutem Aethanol/Aethergemisch und Umkristallisieren daraus erhält man 20 in 87% Ausbeute. Schmelzpunkt = 174°C.

Analyse: $C_{18}H_{19}NO$

| | C | H | N | O |
|---|---|---|---|---|
| Berechnet | 81,48 | 7,21 | 5,27 | 6,02 |
| Gefunden | 80,59 | 7,26 | 5,05 | 7,11 |
| | 80,84 | 7,15 | 4,95 | 7,06 |

Berechnet mit 1/5 Mol Wasser pro Mol Piperidinon:

| | C | H | N | O |
|---|---|---|---|---|
| | 80,40 | 7,22 | 5,21 | 7,14 |

Beispiel 21: Herstellung von trans-4-(4-Methylphenyl)-3-phenyl-2-piperidinon 21

Man rührt 10 g (0,032 Mol) 6 und 60 $cm^3$ Eisessig 4 Stunden lang bei 150°C unter 100 bar Wasserstoffdruck.

Nach Behandlung mit absolutem Aethanol/wasserfreiem Aether 60/40 und Umkristallisieren daraus erhält man 21 in 55% Ausbeute. Schmelzpunkt = 176°C.

Analyse: $C_{18}H_{19}NO$

| | C | H | N | O |
|---|---|---|---|---|
| Berechnet | 81,48 | 7,21 | 5,27 | 6,02 |
| Gefunden | 81,50 | 7,24 | 4,81 | 6,22 |
| | 81,79 | 7,32 | 4,84 | 6,15 |

<u>Beispiel 22:</u> Herstellung von cis-(3-(3,4-Dimethoxyphenyl)-4-phenyl-2-piperidinon 22

Man gibt 5 g (0,014 Mol) 7 und 1 g 5%ige Platinkohle mit 80 cm³ Eisessig in einen Autoklaven. Man erhitzt 5 Stunde n lang auf 60°C unter 30 bar Wasserstoffdruck.

Nach Behandlung gemäss Beispiel 12 und nachfolgendem Umkristallisieren aus absolutem Aethanol erhält man 22 in 70% Ausbeute. Schemlzpunkt = 195-197°C.

<u>Analyse:</u> $C_{19}H_{21}NO_3$

| | C | H | N | O |
|---|---|---|---|---|
| Berechnet | 73,29 | 6,79 | 4,49 | 15,41 |
| Gefunden | 73,26 | 6,67 | 4,65 | 15,42 |

<u>Beispiel 23:</u> Herstellung von trans-3-(3,4-Dimethoxyphenyl)-4-phenyl-2-piperidinon 23

Man gibt 5 g (0,014 Mol) 7 und 1 g 5%ige Platinkohle mit 60 cm³ Eisessig in einen Autoklaven. Man erhitzt 3 Stunden lang unter 80 bar Wasserstoffdruck auf 80°C.

Nach Behandlung gemäss Beispiel 12 und nachfolgendem Umkristallisieren aus absolutem Aethanol/Aether 60/40 erhält man 23 in 78% Ausbeute -Schmelzpunkt = 154°C.

<u>Analyse:</u> $C_{19}H_{21}NO_3$

| | C | H | N | O |
|---|---|---|---|---|
| Berechnet | 73,29 | 6,79 | 4,49 | 15,41 |
| Gefunden | 73,30 | 6,77 | 4,28 | 15,65 |

<u>Beispiel 24:</u> Herstellung von cis-4-(4-Trifluormethylphenyl)-3-phenyl-2-piperidinon 24

Man gibt 7 g (0,019 Mol) 8 und 1,5 g 5%ige Platinkohle mit 75 cm³ Eisessig in einen Autoklaven. Man erhitzt 7 Stunden lang unter 5 bar Wasserstoffdruck auf 40°C. Nach Behandlung gemäss Beispiel 12 und Umkristallisieren aus wasserfreiem Aether/Hexan erhält man 24 in 77% Ausbeute. Schmelzpunkt = 155-156°C.

<u>Analyse:</u> $C_{18}H_{16}F_3NO$

| | C | H | F | N | O |
|---|---|---|---|---|---|
| Berechnet | 67,70 | 5,04 | 17,84 | 4,38 | 5,01 |
| Gefunden | 67,70 | 5,19 | 17,82 | 4,23 | 5,06 (als Differenz) |

Beispiel 25: Herstellung von cis-3-(3,4-Dihydroxyphenyl)-4-phenyl-2-piperidinon 25

Zu einer Lösung von 4 g (0,013 Mol) 22 in 150 $cm^3$ wasserfreiem Methylenchlorid gibt man unter Rühren in einer Stickstoffatmosphäre bei -60°C tropfenweise im Verlauf von 2 Stunden und 30 Minuten eine Lösung von 10 g (0,040 Mol; 3,8 $cm^3$) Bortribromid in 140 $cm^3$ wasserfreiem Methylenchlorid. Man hält die Temperatur 1 Stunde lang bei -60°C. Dann wird 4 Stunden und 30 Minuten lang bei Raumtemperatur weitergerührt.

Man kühlt erneut auf -60°C und setzt dann tropfenweise 100 $cm^3$ wasserfreies Methanol dazu, lässt die Temperatur dann wieder ansteigen, wobei man das Reaktionsgemisch ständig einem starken Stickstoffstrom aussetzt, um den gebildeten Ueberschuss Bromwasserstoffsäure auszutreiben. Nach 1 Stunde Rühren bei Raumtemperatur engt man die Lösung im Vakuum ein $\nu \leq$ 30°C) und löst das so erhaltene Oel in 100 $cm^3$ Methylenchlorid. Das dihydroxylierte Piperidinon wird durch Zusatz von 100 $cm^3$ Wasser unter Rühren ausgefällt. Man filtriert über eine Glasfritte und wäscht mit Methylenchlorid, Natriumbicarbonatlösung und dann mit Wasser neutral. Dabei erhält man 25 in 80% Ausbeute. Schmelzpunkt = 253-255°C.

Analyse: $C_{17}H_{17}NO_3$

| | C | H | N | O |
|---|---|---|---|---|
| Berechnet | 72,07 | 6,04 | 4,94 | 16,94 |
| Gefunden | 71,66 | 6,00 | 4,96 | 17,38 |
| | 71,64 | 6,23 | 4,86 | 17,27 |
| Berechnet mit 0,1 Mol Wasser pro Mol Piperidinon | | | | |
| | 71,61 | 6,07 | 4,91 | 17,39 |

Beispiel 26: Herstellung von trans-3-(3,4-Dihydroxyphenyl)-4-phenyl-2-piperidinon 26

Behandlung von 23 unter den Bedingungen des Beispiels 25 führt zu 26 in 84% Ausbeute. Schmelzpunkt = 235-237°C.

Analyse: $C_{17}H_{17}NO_3$

| | C | H | N | O |
|---|---|---|---|---|
| Berechnet | 72,07 | 6,04 | 4,94 | 16,94 |
| Gefunden | 71,94 | 5,98 | 4,94 | 17,14 |

Beispiel 27: Herstellung von trans-4-(4-Hydroxyphenyl)-3-phenyl-2-piperidinon 27

Behandlung von 13 unter den Bedingungen des Beispiels 25 mit nachfolgendem Umkristallisieren aus absolutem Aethanol führt zu 27 mit 71% Ausbeute. Schmelzpunkt = 233-234°C.

**Analyse:** $C_{17}H_{17}NO_2$

| | C | H | N |
|---|---|---|---|
| Berechnet | 76,38 | 6,41 | 5,24 |
| Gefunden | 76,46 | 6,21 | 5,17 |

Beispiel 28: Herstellung von trans-4-(4-Benzyloxy-phenyl)-3-phenyl-2-piperidinon 28

Man versetzt 1,34 g (0,005 Mol) 27 in Lösung in einem Gemisch aus trockenem Benzol (17 $cm^3$) und Dimethylsulfoxyd (1 $cm^3$) unter einer Stickstoffatmosphäre mit Natriumhydrid (0,3 g NaH 50%ig in Oel, mehrmals mit Benzol gewaschen).

Man rührt das heterogene Gemisch und erhitzt 1 Stunde lang am Rückfluss, kühlt ab und versetzt dann mit einer Lösung von 1,07 g (6,25 . $10^{-3}$ Mol) Benzylbromid in Benzol. Nach 1 Stunde Erhitzen am Rückfluss klärt sich das Gemisch, und es wird bei Raumtemperatur über Nacht weitergerührt.

Man versetzt mit 10 $cm^3$ Wasser und extrahiert mit Methylenchlorid.

Nach Waschen mit wasserfreiem Aether und Umkristallisieren aus Methanol erhält man 28 in 78% Ausbeute. Schmelzpunkt = 196°C.

**Analyse:** $C_{24}H_{23}NO_2$

| | C | H | N |
|---|---|---|---|
| Berechnet | 80,64 | 6,48 | 3,92 |
| Gefunden | 79,73 | 6,25 | 3,85 |
| | 79,56 | | |
| Berechnet mit 0,22 Mol Wasser pro Mol Piperidinon | | | |
| | 79,76 | 6,53 | 3,87 |

Beispiel 29: Herstellung von trans-4-(4-Mesyloxy-phenyl)-3-phenyl-2-piperidinon 29

Man versetzt eine Lösung von 1,34 g (0,005 Mol) 27 in 10 $cm^3$ wasserfreiem Pyridin unter Stickstoff bei 0°C mit 1,14 g (0,01 Mol; 0,77 $cm^3$) Methansulfonylchlorid. Man rührt 1 Stunde lang zwischen 0 und 3°C und giesst das Gemisch dann in 70 $cm^3$ kaltes Wasser. Man extrahiert mit Methylenchlorid und wäscht neutral. Nach Eindampfen und Umkristallisieren aus Methanol erhält man 29 in 61% Ausbeute. Schmelzpunkt = 199°C.

**Analyse:** $C_{18}H_{19}NO_4S$

| | C | H | N |
|---|---|---|---|
| Berechnet | 62,59 | 5,55 | 4,05 |
| Gefunden | 62,47 | 5,62 | 4,03 |

Beispiel 30: Herstellung von 4-Cyan-3-(4-chlor-3-trifluormethylphenyl)-2-phenylbuttersäureäthylester als Gemisch der erythro-und threo-Racemate 30

Man gibt 15 g (0,091 Mol) Phenylessigsäureäthylester, mit 150 $cm^3$ wasserfreiem Aether verdünnt, unter Rühren und unter Stickstoff zusammen mit 4,3 g (0,110 Mol) Natriumamid in einen mittels eines Eis/Salzbads zwischen -12°C und -15°C gehaltenen 500 $cm^3$-Kolben.

Im Verlauf von 45 Minuten versetzt man bei einer Temperatur zwischen -12°C und -15°C mit 20 g (0,086 Mol) 4-Chlor-3-trifluormethylzimtsäurenitril gelöst in 80 $cm^3$ wasserfreiem Tetrahydrofuran. Man lässt die Temperatur auf -5°C anstei -gen und rührt 1 Stunde und 10 Minuten lang, bevor man zwischen -5°C und -10°C mit 50 $cm^3$ Wasser und dann mit 6 n-Salzsäure (ungefähr 40 $cm^3$) bis zur Neutralität hydrolysiert.

Nach Eindampfen zur Trockne gewinnt man quantitativ eine beigefarbene amorphe Verbindung, die hauptsächlich das erythro-Racemat enthält (erythro/threo ≈ 70/30; durch analytische Gaschromatographie auf einer Apiezon-M-Säule bei 200°C und NMR bei 80 MHz in $CDCl_3$, Tripletts, bestimmt).

Nach Umkristallisieren aus Hexan/absolutem Aethanol (70/30) erhält man 26,2 g eines Pulvers, das immer noch ein Gemisch der erythro-und threo-Racemate 30 (e/t ≈ 73/27) enthält. Ausbeute = 76%.

Analyse: $C_{20}H_{17}ClF_3NO_2$

| | C | H | Cl | F | N |
|---|---|---|---|---|---|
| Berechnet | 60,69 | 4,32 | 8,95 | 14,40 | 3,53 |
| Gefunden | 60,62 | 4,73 | 8,88 | 14,72 | 3,46 |

IR (KBr): ν C≡N 2250 $cm^{-1}$, mittel
ν C=O 1710 $cm^{-1}$, stark.

Massenspektrometrie: (70 eV), M/Z (relative Intensität):

397,9 (0,5); 396,9 (2,6); 395,9 (1,9); 395,0 (8,3); 321,9 (10,7); 281,9 (15,0); 164,0 (50,0); 163,0 - (100,0); 135,0 (30,8).

NMR 80 MHz:

$CDCl_3$: Signale bei δ :

7,9-7,0 ppm (8 H, Ar, breiter Komplex).

4,4-3,5 ppm (4 H, breiter Komplex).

{2,9 ppm / 2,4 ppm} 2 H {H4 threo-Komponente, d., $J_{H3-H4}$ = 5 Hz. / H4 erythro-Komponente, schmaler Komplex.}

{1,2 ppm / 1,0 ppm} 3 H {t. threo-Komponente / t. erythro-Komponente.}

Anmerkung: erythro/threo-Verhältnis = 73/27.

$C_6D_6$: Signale bei δ :

7,80-6,70 ppm (8 H, Ar, breiter Komplex).

4,00-3,00 ppm (4 H, breiter Komplex),

2,20 ppm / 1,55 ppm — 2 H — H4, threo-Komponente, schmaler Komplex. / H4, erythro-Komponente, d., $J_{H3-H4}$ = 5 Hz.

0,85 ppm / 0,55 ppm — 3 H — t., threo-Komponente / t., erythro-Komponente.

Beispiel 31: Herstellung von trans-4-(4-Trifluormethylphenyl)-3-phenyl-2-piperidinon 31

Man gibt 15 g (0,041 Mol) erythro-4-Cyan-3-(4-trifluormethylphenyl)-2-phenylbuttersäureäthylester 8 und 3 g 5%ige Platinkohle mit 120 $cm^3$ Eisessig in einen Autoklaven. Man erhitzt unter Stickstoff (-schwacher Ueberdruck) auf 120°C. Dann leitet man Wasserstoff unter hohem Druck (120 bar) ein und rührt 3 Stunden lang. Man kühlt ab, filtriert den Katalysator ab und verdampft die Essigsäure. Das dabei erhaltene Oel wird in Xylol (300 $cm^3$) aufgenommen und 3 Stunden lang am Rückfluss erhitzt. Man verdampft das Xylol und nimmt mit Methylenchlorid auf, um das Gemisch durch Waschen mit gesättigter Natriumbicarbonatlösung und danach destilliertem Wasser zu neutralisieren. Nach Trocknen der organischen Phase über wasserfreiem Natriumsulfat, Filtrieren und Abdampfen des Lösungsmittels erhält man einen viskosen Rückstand, der in wenig Aether kristallisiert. So erhält man 6,5 g (49%) weisse Kristalle, die ungefähr 85% trans-Racemat und 15% cis-Racemat enthalten (durch analytische Hochdruckflüssigkeitschromatographie auf einer Partisil-10-Säule mit Essigester/Methanol 97/3 als Laufmittel bestimmt).

Schnellchromatographie über Kieselsäure (230-400 ASTM-Siebgrösse) unter Verwendung von Essigester als Eluiermittel gestattet es, das trans-Racemat rein zu erhalten.

Nach Umkristallisieren aus Aether/Methanol gewinnt man 5 g (38%) reines 31, Schmelzpunkt = 174-175°C.

Analyse: $C_{18}H_{16}F_3NO$

| | C | H | N | F |
|---|---|---|---|---|
| Berechnet | 67,70 | 5,05 | 4,38 | 17,84 |
| Gefunden | 67,86 | 5,04 | 4,33 | 17,73 |

Massenspektrometrie: (70 eV), M/Z (relative Intensität):

319 (23, $M^+$), 119 (6), 118 (57), 92 (10), 91 (100), 90 (12).

NMR 60 MHz:

$C_6D_6$: Signale bei $\delta$ :

8,80 ppm (1 H, N-H, s.)

7,30-6,30 ppm (9 H, Ar, breiter Komplex)

3,35 ppm (1 H3, d., $J_{H3-H4}$ = 10 Hz).

3,00-2,50 ppm (3 H, breiter Komplex)

1,45-1,10 ppm (2 H, breiter Komplex).

Beispiel 32: Herstellung von trans-4-(4-Chlor-3-trifluormethylphenyl)-3-phenyl-2-piperidinon 32

Man gibt 26 g (0,065 Mol) des Gemischs der Racemate des 4-Cyan-3-(4-chlor-3-trifluormethylphenyl)-2-phenylbuttersäureäthylesters 30 (e/t = 73/27) und 5 g 5%ige Platinkohle mit 180 $cm^3$ Eisessig in einen Autoklaven. Man erhitzt unter Stickstoff (schwacher Ueberdruck) auf 120°C. Dann leitet man Wasserstoff unter hohem Druck (120 bar) ein und rührt 3 Stunden und 30 Minuten lang.

Nach Abfiltrieren des Katalysators, Abdampfen der Essigsäure und Aufnehmen des so erhaltenen viskosen Rückstands in Methylenchlorid wird die organische Phase mit gesättigter Natriumbicarbonatlösung neutralisiert, mit Wasser gewaschen und über wasserfreiem Natriumsulfat getrocknet.

Nach Eindampfen und Ausfällen mit wasserfreiem Aether erhält man ein kristallisiertes Produkt, welches das Gemisch der cis/trans-Racemate im Verhältnis 15/85 enthält (bestimmt durch analytische Hochdruckflüssigkeitschromatographie: Partisil-10-Säule; Essigester/Methanol 97/3).

Schnellchromatographie über Kieselsäure (230-400 ASTM-Siebgrösse) unter Verwendung von Essigester als Eluiermittel ermöglicht es, das trans-Racemat abzutrennen.

Nach Umkristallisieren aus Hexan/absolutem Aethanol 80/20 erhält man 14 g (60%) des reinen trans-Racemats 32.

Schmelzpunkt = 144-146°C.

Analyse: $C_{18}H_{15}ClF_3NO$

| | C | H | Cl | F | N |
|---|---|---|---|---|---|
| Berechnet | 61,11 | 4,26 | 10,02 | 16,11 | 3,95 |
| Gefunden | 61,49 | 4,34 | 9,98 | 16,28 | 3,86 |

IR (KBr): ν C=O 1660 $cm^{-1}$, stark
ν N-H 3190 $cm^{-1}$, mittel.

Massenspektrometrie:(70 eV), M/Z (relative Intensität):

356,0 (1,2); 355,0 (5,8); 354,0 (3,8); 353,0 (18,4); 351,9 (1,2); 118,0 (64,1); 91,0 (71,8); 28,1 (100,0).

NMR 80 MHz:

$C_6D_6$: Signale bei $\delta$ :

8,80 ppm (1 H, s., N-H)

7,30-6,30 ppm (8 H, Ar, breiter Komplex).

3,30 ppm (1 H3, d., $J_{H3-H4}$ = 10 Hz).

3,05-2,40 ppm (3 H, breiter Komplex).

1,50-1,00 ppm (2 H, breiter Komplex).

Beispiel 33: Herstellung von trans-4-(4-Acetoxyphenyl)-3-phenyl-2-piperidinon 33

Man versetzt 2,67 g (0,01 Mol) trans-4-(4-Hydroxyphenyl)-3-phenyl-2-piperidinon 27, gelöst in 20 $cm^3$ wasserfreiem Pyridin, unter Stickstoff bei 0°C mit 2 $cm^3$ (0,028 Mol) Acetylchlorid. Man rührt 2 Stunden und 30 Minuten lang bei 0° und dann 48 Stunden lang bei Raumtemperatur. Das Reaktionsgemisch wird dann auf 50 g Eis gegossen. Man gibt eine Spatelspitze Natriumbicarbonat dazu, rührt und extrahiert dann mit Methylenchlorid (3-mal 60 $cm^3$).

Die organische Phase wird nacheinander mit Wasser, verdünnter Salzsäure und mit Wasser neutral gewaschen und dann über wasserfreiem Natriumsulfat getrocknet. Nach Abfiltrieren und Abdampfen des Lösungsmittels erhält man 2,6 g -(84%) bröckligen weissen Schaum, der aus Methanol umkristallisiert wird. Nach Waschen mit Aether und Trocknen erhält man 1,65 g (53%) der trans-Verbindung 33 in Form weisser Kristalle vom Schmelzpunkt 201-204°C.

Analyse: $C_{19}H_{19}NO_3$

| | C | H | N |
|---|---|---|---|
| Berechnet | 73,76 | 6,19 | 4,52 |
| Gefunden | 73,98 | 6,14 | 4,40 |
| | | 6,36 | |

IR (KBr): ν N-H 3340 $cm^{-1}$, schwach
ν C=O 1740 $cm^{-1}$, stark (Ester)
ν C=O 1650 $cm^{-1}$, stark (Lactam).

Massenspektrographie: (70 eV); M/Z (relative Intensität):

311,0 (1,1); 310,0 (6,6); 309,0 (16,8; $M^{+\cdot}$); 267,1 - (35,9); 176,1 (5,1); 165,1 (6,2); 147,0 (19,1); 133,0 - (38,0); 120,0 (25,5); 119,0 (12,6); 118,0 (100); 107,0 (5,0); 91,0 (14,4); 89.9 (16.2); 89,0 (6,0).

Beispiel 34: Herstellung von trans-3-Phenyl-4-(4-phenoxyphenyl)-2-piperidinon 34

Man erhitzt 6,2 g (0,023 Mol) trans-4-(4-Hydroxyphenyl)-3-phenyl-2-piperidinon 27, 8,6 g (0,023 Mol) Diphenyljodoniumbromid und eine Lösung von 0,94 g Natriumhydroxyd in 170 $cm^3$ doppelt destilliertem Wasser 24 Stunden lang unter Rühren zum Rückfluss. Man kühlt ab und extrahiert mit Methylenchlorid. Die organische Phase wird nacheinander mit 5%iger Natronlauge und dann mit Wasser neutral gewaschen und über wasserfreiem Natriumsulfat getrocknet. Nach Abfiltrieren und Eindampfen erhält man 6 g Feststoff, der aus Methanol umkristallisiert wird. Dies ergibt 5,3 g (66%) reines 34 vom Schmelzpunkt 137-138°C.

Analyse: $C_{23}H_{21}NO_2$

| | C | H | N |
|---|---|---|---|
| Berechnet | 80,44 | 6,16 | 4,07 |
| Gefunden | 80,43 | 5,90 | 4,04 |

IR (KBr): ν N-H 3200 $cm^{-1}$, mittel
ν C=O 1660 $cm^{-1}$, stark
ν C-O-C 1240 $cm^{-1}$, stark.

Massenspektrometrie: (70 eV); M/Z (relative Intensität):

345,0 (2,4); 344,0 (17,5); 343,0 (63,2; $M^{+\cdot}$); 210,0 - (9,3); 209,0 (56,4); 197,0 (10,9); 196,0 (65,1); 178,0 (7,5); 167,0 (5,2); 165,0 (8,8); 152,0 (5,2); 147,0 - (19,7); 119,0 (10,7); 118,0 (100,0); 116,0 (20,5); 115,0 (15,6); 91,0 (14,1); 89,9 (19,9); 89,0 (5,7); 77,1 (21,2); 65,1 (5,1).

Beispiel 35: Herstellung von trans-4-(4-Aethoxyphenyl)-3-phenyl-2-piperidinon 35

Man versetzt eine Lösung von 6,7 g (0,025 Mol) trans-4-(4-Hydroxyphenyl)-3-phenyl-2-piperidinon 27 und 1,3 g Natriumhydroxyd in Plättchenform in 50 $cm^3$ Wasser unter fortgesetztem Rühren tropfenweise bei 0°C mit 3,6 $cm^3$ - (0,027 Mol; 4,23 g) Diäthylsulfat. Nach 5 Stunden unter Rückfluss (92°C) kühlt man ab und extrahiert die so erhaltene Suspension mit Methylenchlorid. Die organische Phase wird nacheinander mit verdünnter Natronlauge und dann mit Wasser neutral gewaschen und über Natriumsulfat getrocknet. Dabei erhält man 5,9 g (80%) weissen Schaum, der aus Aethanol/ Aether (70/30) umkristallisiert wird. Dabei gewinnt man 5 g (67%) reines 35 vom Schmelzpunkt 161-162°C (Gestaltänderung bei 153°C).

Analyse: $C_{19}H_{21}NO_2$

| | C | H | N |
|---|---|---|---|
| Berechnet | 77,26 | 7,16 | 4,74 |
| Gefunden | 77,13 | 7,20 | 4,79 |

Massenspektrographie: (70 eV); M/Z (relative Intensität):

297,1 (1,1); 296,1 (8,5); 295,1 (43,2; $M^{+\cdot}$); 162,1 - (10); 161,0 (79,1); 149,0 (6,3); 148,0 (52,6); 147,0 - (13,6); 133,0 (17,4); 120,0 (30,0); 119,0 (14,0); 118 - (100,0); 115,0 (5,4); 91,0 (14,8); 89,9 (20,1); 89,0 - (5,6).

Beispiel 36: Herstellung von erythro-4-Cyan-3-(4-dimethylaminophenyl)-2-phenylbuttersäureäthylester 36

1.

Herstellung von trans-4-Dimethylamino-zimtsäurenitril
In einen27,65 g (0,200 Mol) Kaliumcarbonat, 35 $cm^3$ Wasser und 21,25 g (0,119 Mol) Diäthyl-cyanmethylphosphonat enthaltenden Kolben gibt man 14,92 g (0,100 Mol) 4-Dimethylaminobenzaldehyd, in ganz wenig Tetrahydrofuran gelöst. Nach 5 Stunden unter Rückfluss (72°C) kühlt man ab und versetzt mit 200 $cm^3$ Wasser. Der dabei erhaltene gelbe Niederschlag enthält im wesentlichen das trans-Isomer. Nach Umkristallisieren aus Aethanol bei 95° erhält man 11 g - (64%) reines trans-4-Dimethylamino-zimtsäurenitril vom Schmelzpunkt 168-169°C.

2. Zu einem Gemisch aus 9 g (0,052 Mol) trans-4-Dimethylamino-zimtsäurenitril in 130 $cm^3$ wasserfreiem Aether und 2,6 g (0,066 Mol) Natriumamid gibt man unter Rühren im Verlauf von 25 Minuten bei 0°C eine Lösung von 9,1 g (0,055 Mol) Phenylessigsäureäthylester in 40 $cm^3$ Aether. Nach beendeter Zugabe lässt man das Gemisch auf Raumtemperatur kommen und rührt noch 1 Stunde weiter, bevor man es über Nacht stehen lässt. Das Reaktionsgemisch wird dann mit 5 $cm^3$ Wasser und 30 $cm^3$ 4 n-Salzsäure bis zur Neutralität hydrolysiert und dann mit Methylenchlorid extrahiert. Die organische Phase wird mit Wasser gewaschen und dann über wasserfreiem Natriumsulfat getrocknet. Nach Filtrieren und Abdampfen gewinnt man einen braunen Feststoff, der ein erythro/threo-Gemisch im Verhältnis 95/5 enthält. Durch Umkristallisieren aus absolutem Aethanol erhält man 14,1 g (80%) reine erythro-Verbindung 36 vom Schmelzpunkt 141°C.

Beispiel 37: Herstellung von trans-4-(4-Dimethylaminophenyl)-2-piperidinon 37

Man gibt 11,3 g der reinen erythro-Verbindung 36, 2 g Platinkohle (5% Pt/C) und 50 $cm^3$ Eisessig in einen 250 $cm^3$-Autoklaven. Man erhitzt auf 120°C, wobei der Temperaturanstieg unter - schwachem Stickstoffüberdruck erfolgt. Wasserstoff unter hohem Druck (150 bar) wird erst eingeleitet, nachdem sich die Temperatur stabilisiert hat. Nach üblicher Behandlung gewinnt man eine beigefarbenes Oel, löst dieses in Xylol, erhitzt das Ganze 4 Stunden lang am Rückfluss und dampft das Xylol dann ab. Der Rückstand wird in Methylenchlorid gelöst. Die organische Phase wird mit gesättigter Natriumbicarbonatlösung neutralisiert, dann mit Wasser gewaschen und über wasserfreiem Natriumsulfat getrocknet. Nach Eindampfen erhält man 9 g (92%) eines beigefarbenen Feststoffs, der ein trans/cis-Gemisch im Verhältnis 93/7 enthält. Umkristallisieren des Rohprodukts aus Aethanol gestattet es nicht, das reine trans-Diastereoisomer zu isolieren; dieses wird durch "flash"-Chromatographie rein gewonnen:

-Kieselgelsäule 230-400 ASTM-Siebgrösse,

-Laufmittel: Chloroform/Aethanol 99/1.

Man erhält 7 g (71%) der trans-Verbindung 37 vom Schmelzpunkt 218-219°C durch Umkristallisieren aus absolutem Aethanol.

Analyse: $C_{19}H_{22}N_2O$

| | C | H | N | O |
|---|---|---|---|---|
| Berechnet | 77,51 | 7,53 | 9,51 | 5,43 |
| Berechnet (0,175 Mol $H_2O$) | 76,69 | 7,56 | 9,41 | 6,31 |
| Gefunden | 76,68 | 7,39 | 9,15 | 6,39 |

Massenspektrographie: (70 eV); M/Z (relative Intensität):

295,1 (9,6); 294,1 (47,3; M $^{+\cdot}$); 161,0 (11,9); 160,0 - (100,0); 148,1 (13,1); 147,1 (39,9); 146,1 (14,1); 118,0 (14,2); 90,0 (5,1).

Beispiel 38: Herstellung von erythro-4-Cyan-3-(2,4-dichlorphenyl)-2-phenyl-buttersäureäthylester 38

1.

Herstellung von trans-2,4-Dichlor-zimtsäurenitril.
Man versetzt eine Lösung von 8 g Kaliumcarbonat in 10 $cm^3$ Wasser mit 6 g (0,034 Mol) Diäthyl-cyanmethylphosphonat und dann mit 5 g 2,4-Dichlorbenzaldehyd, gelöst in 10 $cm^3$ wasserfreiem Tetrahydrofuran. Man rührt 10 Minuten lang bei Raumtemperatur und versetzt mit 20 $cm^3$ Wasser. Der gebildete Niederschlag wird mit Wasser neutral gewaschen (8-mal 20 $cm^3$ Wasser) und dann mit Aether/Hexan gewaschen. Dabei erhält man 3,7 g (74%) 2,4-Dichlor-zimtsäurenitril in Form weisser Kristalle vom Schmelzpunkt 156°C.

2. Man gibt 23 g (0,14 Mol) Phenylessigsäureäthylester, gelöst in 250 $cm^3$ wasserfreiem Aether, unter Rühren und unter Stickstoff zusammen mit 6 g (0,153 Mol) Natriumamid in einen mittels Eis auf 0°C gehaltenen 1-Liter Kolben und versetzt dann im Verlauf von 30 Minuten mit 25 g (0,126 Mol) trans-2,4-Dichlor-zimtsäurenitril in 350 $cm^3$ wasserfreiem Tetrahydrofuran. Nach 6 Stunden Rühren bei 0°C wird das Gemisch mit 20 $cm^3$ Wasser und dann 6 n-HCl hydrolysiert, bis Neutralität erreicht ist. Nach Eindampfen und Auflösen des Rückstands in Methylenchlorid wäscht man die organische Phase mit Wasser und trocknet über wasserfreiem Natriumsulfat. Das erhaltene Rohprodukt enthält nur das erythro-Racemat. Es wird aus absolutem Aethanol umkristallisiert, was 32 g (70%) erytrho-38 in Form weisser Kristalle vom Schmelzpunkt 95-96°C ergibt.

Analyse: $C_{19}H_{17}Cl_2CO_2$

| | C | H | Cl | N |
|---|---|---|---|---|
| Berechnet | 62,99 | 4,72 | 19,57 | 3,86 |
| Gefunden | 62,93 | 4,86 | 19,85 | 3,76 |

IR (KBr): ν C≡N 2220 $cm^{-1}$, mittel
ν C=O 1730 $cm^{-1}$, stark.

Massenspektrometrie: (70 eV); M/Z (relative Intensität):

365,1 (0,9; M $^{+\cdot}$); 364,0 (1,0); 363,0 (4,6; M $^{+\cdot}$); 362,0 (1,6); 361,0 (6,7; M $^{+\cdot}$); 326,1 (6,2); 289,9 (7,6); 288,0 (11,5); 250,0 (8,2); 248,0 (12,4); 213,0 (9,4); 199,9 (14,0); 198,0 (21,6); 179,1 (5,0); 178,1 (28,7); 177,1 (8,0); 176,1 (8,8); 165,0 (12,5); 164,1 (100); 163,0 (91,3); 136 (13,8); 135 (39,3); 118 (7,2); 107 - (27,8); 106 (6,4); 91,0 (34,9); 89,9 (5,9); 89,0 (6,7); 88,0 (7,0); 79,0 (14,6); 77,1 (7,6).

Beispiel 39: Herstellung von threo-4-Cyan-3-(2,4-dichlorphenyl)-2-phenylbuttersäureäthylester 39

Durch Epimerisierung des erythro-Isomeren 38 in alkalischem Milieu (äthanolische 0,1 m-Kalilauge) und nachfolgende Abtrennung durch flash-Chromatographie (Kieselgel, Siebgrösse 230-400, Laufmittel: Hexan/Essigester 90/10) lässt sich das reine threo-Isomer 39 in Form von Kristallen vom Schmelzpunkt 78-79°C erhalten.

Beispiel 40: Herstellung von trans-3-(2,4-Dichlorphenyl)-2-piperidinon 40

Schmelzpunkt: 170 -171°C (aus Ethanol umkristallisiert).

Pharmakologische Versuchsberichte

I -Fall der Piperidinone

Die Untersuchungen der akuten Toxizität an der Maus, der Motoraktivität an der Maus, der Stereotypien an der Ratte, der Antireserpinaktivität an der Maus, der Antiapomorphinaktivität an der Maus, der Antioxotremorinaktivität an der Maus, der Wirkung auf die Toxizität des Yohimbins an der Maus und der Wechselwirkung mit Natriumbarbital und Pentobarbital lassen sich wie folgt gliedern:

1 -Wirkungen in bestimmten Tests

-Höhere Toxizität bei gruppierten Mäusen

-Erhöhung der Motoraktivität an der Maus

-Stereotype Bewegungen an der Ratte

-Antagonismus gegen durch Natriumbarbital induzierten Schlaf an der Maus

-Antagonismus gegen durch Reserpin induzierte Hypothermie

-Antagonismus gegen durch Apomorphin -(hohe Dosis) induzierte Hypothermie

-Antagonismus gegen durch Oxotremorin induzierte Hypothermie

-Leichte Potenzierung der Toxizität des Yohimbins

2 -Abwesenheit von Wirkungen in anderen Tests

-Kein Antagonismus gegen durch Pentobarbital induzierten Schlaf

-Kein Antagonismus gegen durch Reserpin provozierte Ptose und Akinesie

-Keine Aenderung des durch Apomorphin provozierten Aufrichtens und Stereotypien

-Kein Antagonismus gegen durch Oxotremorin provozierten Tremor bzw. periphere Symptome.

Gemäss diesem Profil kann man das erfindungsgemässe Arzneimittel bei den ein doppeltes Spektrum besitzenden Substanzen eingliedern: Psychostimulanz und Antidepressivum.

1) Stimulanzartige Wirkung, gekennzeichnet durch eine höhere Toxizität bei gruppierten Mäusen, Zunahme der Motoraktivität in der Maus, einige stereotype Bewegungen an der Ratte und Antagonismus gegen durch Natriumbarbital induzierten Schlaf an der Maus.

2) Antidepressive Wirkung. Das erfindungsgemässe Produkt zeigt Wirkungen in den hauptsächlichen Tests, die eine antidepressive Aktivität reflektieren:

-Antagonistische Wirkung auf durch Reserpin induzierte Hypothermie

-Antagonistische Wirkung auf durch Apomorphin (hohe Dosis) induzierte Hypothermie

-Antagonistische Wirkung auf durch Oxotremorin induzierte Hypothermie

-Leichte potenzierende Wirkung auf die Toxizität des Yohimbins.

Für die 3 unten aufgezählten Produkte ermöglichten es die verschiedenen Tests, die kleinste wirksame Dosis (mg/kg) zu bestimmen, wie in der nachfolgenden Tabelle angegeben:

TABELLE

AKTIVITAETSANGABEN:

| Beispiel | Psychostimulierend | | | | | Antidepressiv | | | |
|---|---|---|---|---|---|---|---|---|---|
| | Toxizität | Aktimetriemessungen | Stereotypie | Hypnotisch | | Hypothermie | | | Yohimbintoxizität |
| | | | | Barbitalantagonismus | Pentobarbital | Reserpin | Apomorphin | Oxotremorin | |
| Nr. 22 | 64 | 0,25 | 4 | 1 (p.o.) | - | 0,25 | 0,5 | - | 0,5 (i.p.) |
| Nr. 23 | 256 | 2 | 4 | 4 (p.o.) | - | 2 | 4 (i.p. p.o.) | - | 4 (i.p.) |
| Nr. 32 | >256 | an Mäusen nicht geprüft | - | 32 | Potenzierung 32 | 1 | 1 | 4 | 2 |

Beschreibung der angewandten pharmakologischen Methoden

Die pharmakologischen Prüfungen werden an männlichen Ratten mit einem Gewicht zwischen 170 und 220 g (Wistar-Stamm) und männlichen Mäusen mit einem Gewicht zwischen 18 und 23 g - (NMRI-Stamm) durchgeführt.

Alle Versuche werden blind ausgeführt (wobei der Experimentator bei der Durchführung der Prüfungen nicht weiss, welche Tiere die als aktiv angenommene Substanz in welcher Dosis erhalten haben).

Falls nicht anders angegeben werden 10 Tiere pro Gruppe eingesetzt.

Sämtliche Versuche werden in einem Laboratorium bei konstant gehaltener Temperatur (22 ± 1 °C) durchgeführt.

Die Verabreichungen erfolgten entweder oral oder intraperitoneal.

Die Injektionsvolumina betragen 0,5 ml pro 100 g Körpergewicht an der Ratte und 0,25 ml pro 20 g Körpergewicht bei der Maus.

Bestimmung der akuten Toxizität an der Maus

Die $DL_{50}$ wird nach Behrens und Karber bestimmt.

Akute Toxizität an der isolierten Maus und an Mäusegruppen

Die Mäuse (10 pro Gruppe) werden einzeln - (Toxizität an der isolierten Maus) oder in Gruppen von 10 (spezielle Toxizität) in Kästchen der Abmessungen 20 x 10 x 10 cm plaziert.

Die Mortalität wird nach 1, 2, 3, 4 und 24 Stunden bestimmt.

Motoraktivität an der Maus

Die Motoraktivität wird mittels Photozellenaktimetern bestimmt (Boissier und Simon, Arch. Int. Pharmacodyn. 1965, 158, 212-221).

Die Mäuse (10 bzw. Vielfache von 20 pro Gruppe) werden gleich nach der Verabreichung der zu untersuchenden Verbindungen in das Aktimeter plaziert.

Die Motoraktivität wird nach dem Einsetzen in die Aktimeter bis zur 60. Minute gemessen. Die Zähler werden nach 30 Minuten abgelesen, dann auf 0 zurückgestellt und alle 10 Minuten während 30 Minuten abgelesen.

Die Motilität der mit den verschiedenen Verbindungen behandelten Tiere wird mit derjenigen einer destilliertes Wasser erhaltenen Kontrollgruppe verglichen.

Antireserpinaktivität

Zum Zeitpunkt 0 erhalten die Mäuse Reserpin (2,5 mg/kg) auf intraperitonealem Weg.

Nach 4 Stunden, wenn die Hypothermie als genügend erachtet wird (30 bis 33°C), verabreicht man die verschiedenen zu untersuchenden Verbindungen. Die Rektaltemperatur wird alle 30 Minuten während 2 Stunden mit einer ein Thermoelement tragenden Sonde gemessen, die aufkonstante Tiefe eingeschoben wird. Die Rektaltemperatur der mit den verschiedenen erfindungsgemässen Verbindungen behandelten Tiere wird mit der Temperatur in einer Kontrollgruppe verglichen, die destilliertes Wasser auf dem gleichen Weg wie die zu untersuchenden Verbindungen erhält.

Die Akinesie (Bewegungsarmut) und Ptose - (Oeffnungsgrad der Augenlids von 0 bis 4 nach Rubin und Mitarbeiter, 1957) werden ebenfalls 2 Stunden lang alle 30 Minuten bewertet.

Die eine antidepressive Aktivität besitzenden Produkte wirken antagonistisch gegen die durch Reserpin induzierte Hypothermie. Für jede Dosis der verabreichten Verbindung berechnet man die prozentuale Hemmung der Hypothermie.

Antiapomorphinaktivität

Zum Zeitpunkt 0, nach Ausgleich der Rektaltemperaturen, werden die Mäuse (6 pro Gruppe) in Einzelkästen (11 x 3,5 x 4 cm) plaziert, in denen sie beobachtbar sind. 30 Minuten später erhalten sie Apomorphin in einer Dosis von 16 mg/kg auf subkutanem Weg. Die Rektaltemperatur wird 30 Minuten nach dem Apomorphin gemessen. Kontrolltiere erhalten destilliertes Wasser statt der zu untersuchenden Verbindungen. Die bei diesen Kontrolltieren, die 16 mg/kg Apomorphin erhalten haben, beobachtete Hypothermie beträgt im Durchschnitt 33°C. Für jede Dosis der Verbindungen berechnet man die prozentuale Hemmung der Hypothermie.

Antioxotremorinaktivität

Nach Ausgleich der Rektaltemperaturen erhalten Mäuse (6 pro Gruppe) entweder destilliertes Wasser oder die verschiedenen zu untersuchenden Verbindungen.

30 Minuten später verabreicht man Oxotremorin in einer Dosis von 0,5 mg/kg auf intraperitonealem Weg.

Dann werden die Mäuse beobachtet: der Tremor wird je nach seiner. Intensität von 0 bis 3 bewertet, und die Akinesie wird alle 10 Minuten während 30 Minuten als anwesend oder abwesend bestimmt.

Ausserdem beobachtet man 2 Stunden lang das Auftreten peripherer Symptome (Tränenfluss, Darmentleerung). Die Rektaltemperatur wird alle 30 Minuten während 2 Stunden nach der Oxotremoringabe gemessen.

Die eine antidepressive Aktivität besitzenden Produkte wirken antagonistisch gegen die durch Oxotremorin induzierte Hypothermie. Die anticholinergen Substanzen wirken antagonistisch gegen die peripheren Symptome und Tremor.

Für jede Dosis der Verbindung berechnet man die prozentuale Hemmung der Hypothermie.

Wirkung auf die Toxizität des Yohimbins

Zum Zeitpunkt 0 erhalten die Mäuse entweder destilliertes Wasser oder verschiedene zu untersuchende Verbindungen. Nach 30 Minuten verabreicht man auf subkutanem Weg eine subtoxische Dosis von 25 mg/kg Yohimbinhydrochlorid.

Die Letalität wird 4 Stunden lang jede Stunde verfolgt und dann nach 24 Stunden verzeichnet.

Die eine antidepressive Aktivität besitzenden Produkte führen zu einer Potenzierung der Toxizität des Yohimbins.

Wechselwirkung mit Natriumbarbital bzw. Pentobarbital

Die Tiere (6 Mäuse pro Gruppe) werden in individuelle durchsichtige, 20 x 10 x 10 cm grosse Kunststoffkästen plaziert und erhalten die zu untersuchenden Verbindungen oder destilliertes Wasser 30 Minuten vor einer hypnotischen Dosis Natriumbarbital (180 mg/kg intraperitoneal) bzw. Pentobarbital (5 mg/kg intraperitoneal). Die Zeitpunkte der Unterdrückung und des Wiedererscheinens des Aufrichtreflexes werden für jedes Tier notiert.

II -Fall der Ester-nitrile

Das erfindungsgemässe Produkt zeigt eine starke und anhaltende nierengefässerweiternde Wirkung, die sich in vitro an der isolierten Rattenniere und in vivo an der narkotisierten Ratte (Pentobarbital) zeigen lässt. An der perfundierten isolierten Niere (nach Schmidt und Imbs, J. Cardiovasc. Pharmacol. 2, 595-605, 1980), führt das erfindungsgemässe Produkt (Beispiel 3) in einer Konzentration von 3 . $10^{-5}$ M zu einer Erniedrigung des Gefässwiderstands um 50% der gefässerweiternden Wirkung von $10^{-4}$ M Papaverin. Bei Verabreichung über eine Magensonde in einer Dosis von 1 mg/kg an der narkotisierten Ratte unter kontinuierlicher Messung des Renalis-und Iliacadurchflusses (elektromagnetische Durchflussmessung) und des arteriellen Drucks führt das erfindungsgemässe Produkt (Beispiel 3) zu einer Erniedrigung des Nierengefässwiderstands um durchschnittlich 35 ± 2% (von 42,6 ± 0,7 auf 27,8 ± 0,6 mmHg. min.$ml^{-1}$; m ± Standardfehler des Mittelwerts, n = 10) die nach der Sondeneinführung 4 Stunden lang anhält, ohne dass sich der Dünndarmgefässwiderstand oder der arterielle Druck ändert. Bei Dosierungen von 100 oder 10 mg/kg (Magensonde) führt das erfindungsgemässe Produkt (Beispiel 3) zu einer dosisabhängigen Dünndarmgefässverengung, die bei einer Dosis von 1 mg/kg verschwindet.

Diese Wirkung auf das Nierengefässbett lässt sich zur Behandlung akuter und chronischer Niereninsuffizienz sowie zur Langzeitbehandlung von Bluthochdruck ausnutzen.

Die erfindungsgemässen Arzneimittel können für sich oder zusammen mit anderen Arzneimitteln und in allen üblichen galenischen Formen verabreicht werden. Die orale Verabreichungsform wird bevorzugt.

Die nützliche, oral verabreichte Tagesdosis liegt zwischen 25 und 250 mg. Die verabreichte galenische Form wird zwischen 15 und 100 mg, vorzugsweise 50 mg, dosiert.

Wie aus dem obigen hervorgeht, ist die Erfindung keineswegs auf solche Ausführungs-und Anwendungsformen beschränkt, die oben mehr im einzelnen beschrieben sind; im Gegenteil umfasst sie sämtliche Varianten, die ein Fachmann ins Auge fassen kann, ohne den Rahmen oder den Umfang der vorliegenden Erfindung zu verlassen.

## Ansprüche

1. Neue Arzneimittel, bestehend aus 2,3-oder 3,4-Diphenyl-γ-nitril-esterderivaten der Formeln A oder B

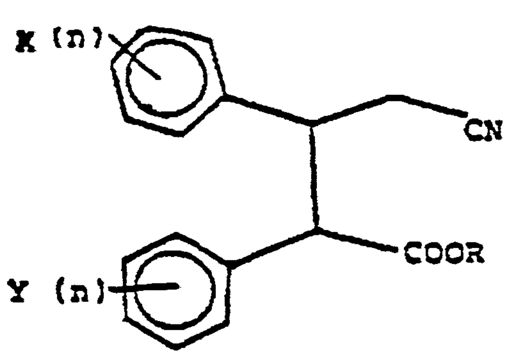

(A)

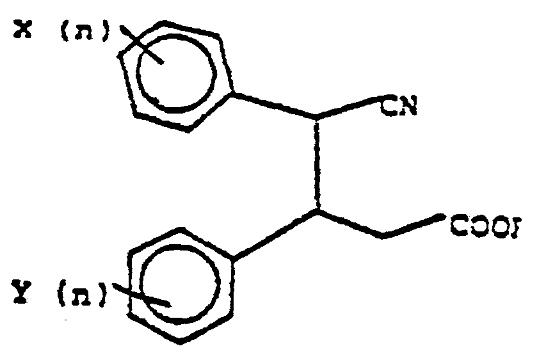

(B)

worin R für ein Wasserstoffatom, ein pharmazeutisch unbedenkliches Alkali-oder Erdalkalimetall oder eine $C_1$-$C_4$-Alkylgruppe sowie X und Y, die gleich oder verschieden sein können, für ein Wasserstoff- oder Halogenatom oder eine $C_1$-$C_4$-Alkyl-, $C_1$-$C_4$-Alkoxy-, Phenyl-, Phenyl-$C_1$-$C_4$-alkyl-oder Trifluormethylgruppe stehen, wobei n 1 bis 3 beträgt, falls X und/oder Y von Wasserstoff verschieden sind, oder aus Ringschlussprodukten der 2,3-Diphenylderivate, der Formel C

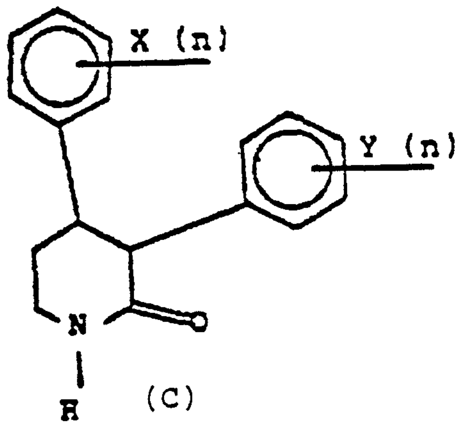

(C)

worin R für ein Wasserstoffatom, ein pharmazeutisch unbedenkliches Alkali-oder Erdalkalimetall oder eine $C_1$-$C_4$-Alkylgruppe sowie X und Y, die gleich oder verschieden sein können, für ein Wasserstoff- oder Halogenatom oder eine Trifluormethyl-, $C_1$-$C_4$-Alkyl-, Phenyl-, Phenyl-$C_1$-$C_4$-alkyl-, $C_1$-$C_4$-Alkoxy-, Hydroxyl-, Acyloxy-, Phenyloxy-, Benzyloxy-, $C_1$-$C_4$-Alkylsulfonyl-oder Di-$C_1$-$C_4$-alkylaminogruppe stehen, wobei n 1 bis 3 beträgt, falls X und/oder Y von Wasserstoff verschieden sind, oder enthaltend diese Verbindungen.

2. Arzneimittel nach Anspruch 1, dadurch gekennzeichnet, dass die durch die Formeln A und B dargestellten Produkte die erythro-oder threo-bzw. im Fall der Piperidinone (Formel C) die cis-oder trans-Konfiguration besitzen.

3. Ringschlussverfahren für 2,3-Diphenyl-γ-nitrilesterderivate der Formel C gemäß Anspruch 1 unter Festlegung der Stereochemie der entstehenden Piperidinone, dadurch gekennzeichnet, dass man, wenn ein reines cis-Derivat erwünscht ist, von einem reinen erythro-Produkt ausgeht und dieses bei einem Druck zwischen 10 und 80 bar und einer Temperatur zwischen 30 und 100°C hydriert bzw., wenn ein reines trans-Derivat erwünscht ist, entweder von einem reinen threo-Derivat oder einem erythro-Derivat ausgeht, das bei einem Druck zwischen 80 und 180 bar und einer Temperatur zwischen 80 und 180°C hydriert wird.

4. Neue 2,3-Diphenyl-γ-nitril-esterderivate der nachfolgenden Formeln I bis VIII:

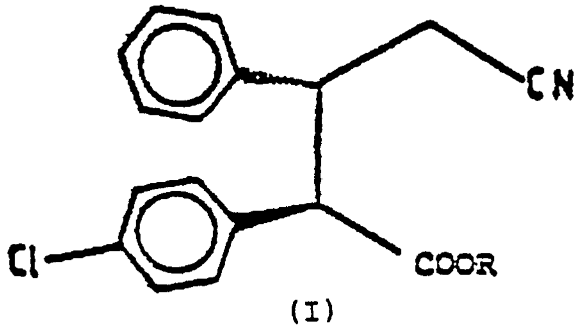

(I)

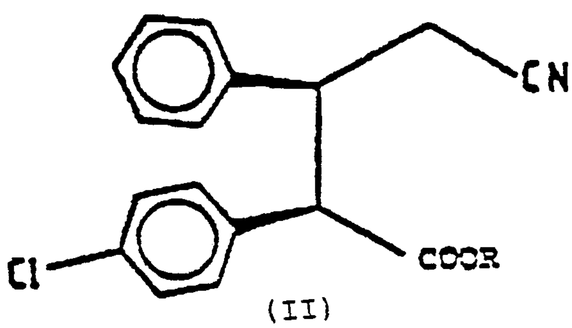

(II)

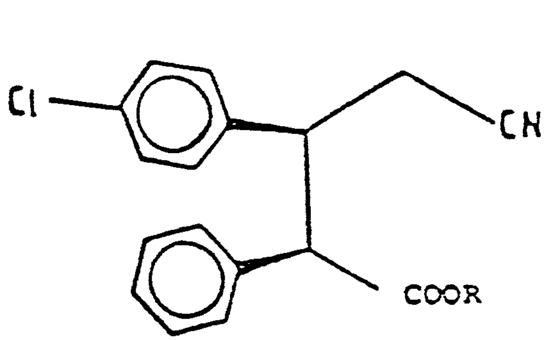

(III)

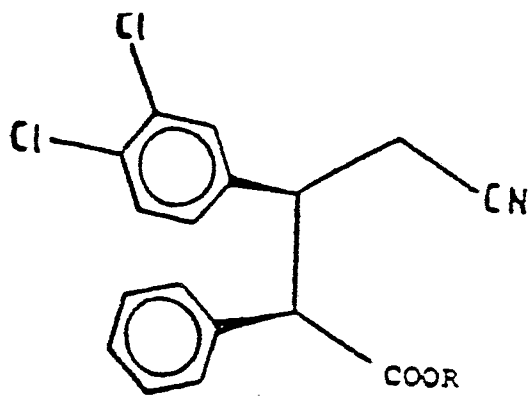

(IV)

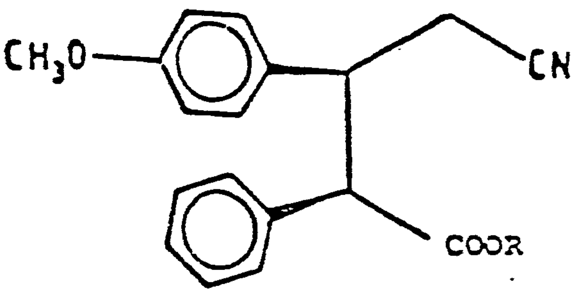

(V)

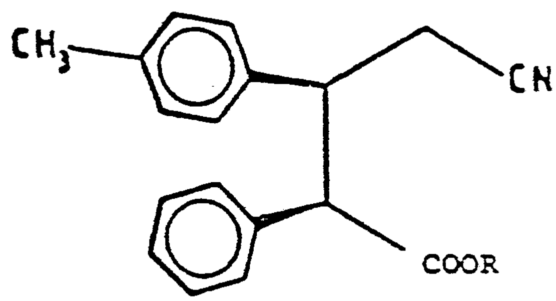

(VI)

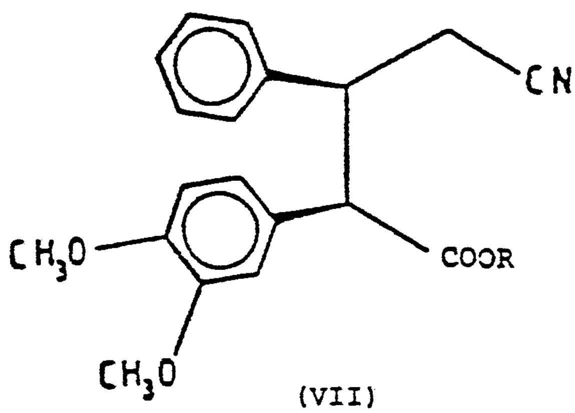

(VII)

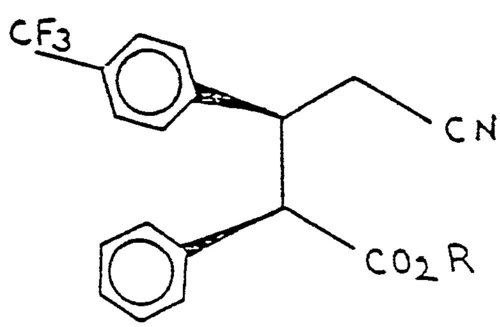

(VIII)

worin R für ein Wasserstoffatom, ein pharmazeutisch unbedenkliches Alkali-oder Erdalkalimetall oder eine $C_1$-$C_4$-Alkylgruppe steht.

5. Neue 3,4-Diphenyl-γ-nitril-esterderivate der Formeln IX und X

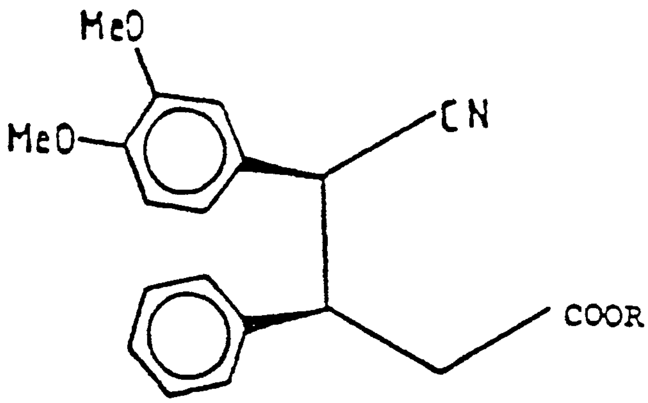

(IX)

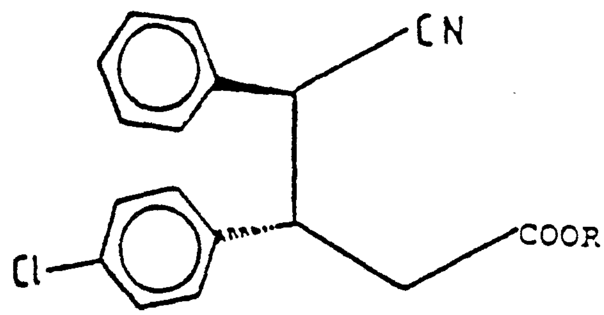

(X)

worin R für ein Wasserstoffatom, ein pharmazeutisch unbedenkliches Alkali-oder Erdalkalimetall oder eine $C_1$-$C_4$-Alkylgruppe steht.

6. Neue 3,4-Diphenyl-piperidinone der nachfolgenden Formeln XI bis XXVII:

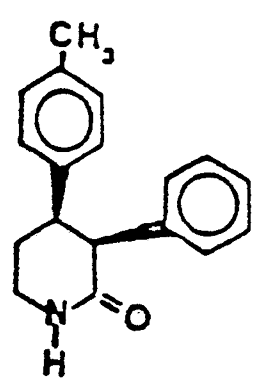

(XI)

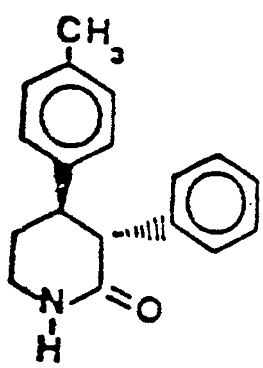

(XII)

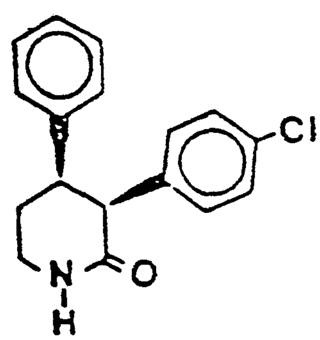

(XIII)

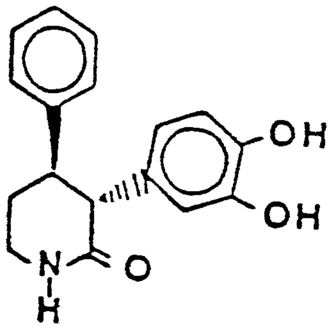

(XIV)

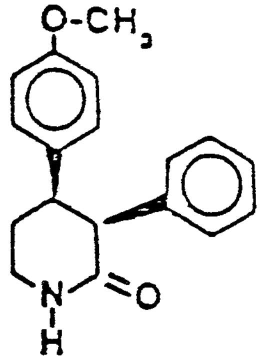

(XV)

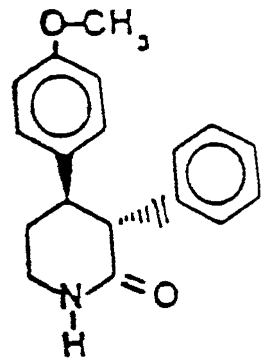

(XVI)

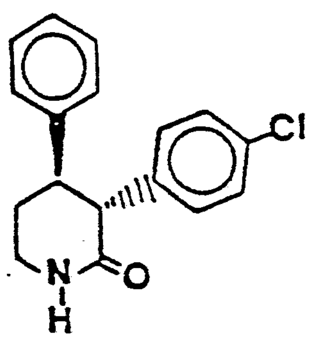

(XVII)

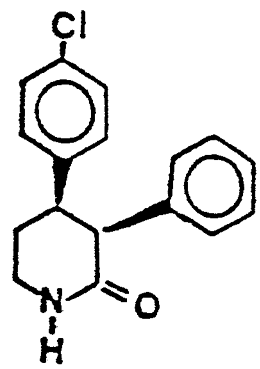

(XVIII)

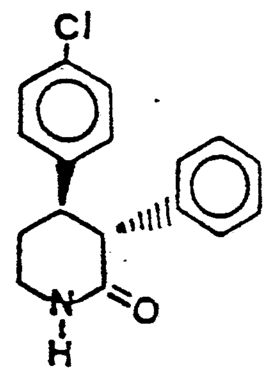

(XIX)

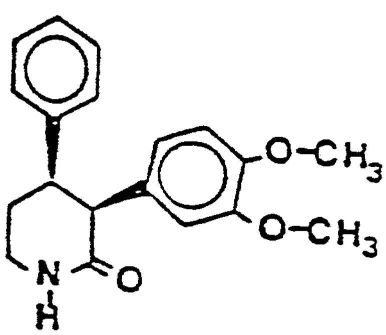

(XX)

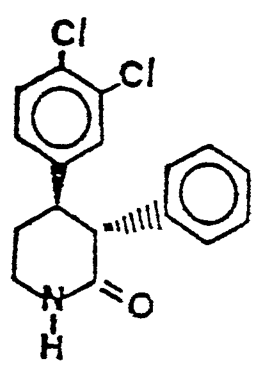

(XXI)

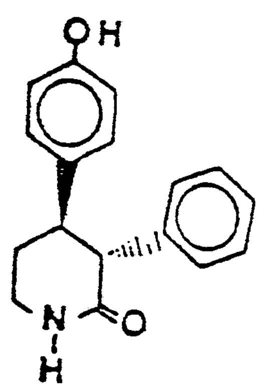

(XXII)

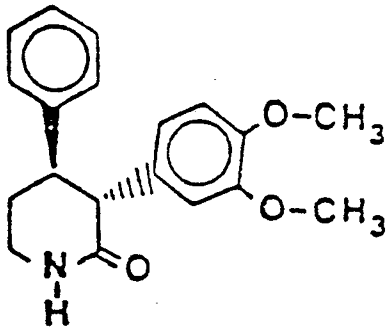

(XXIII)

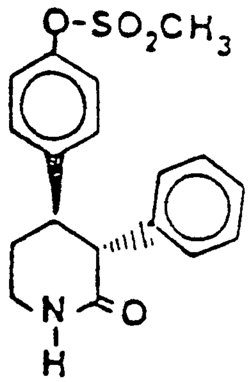

(XXIV)

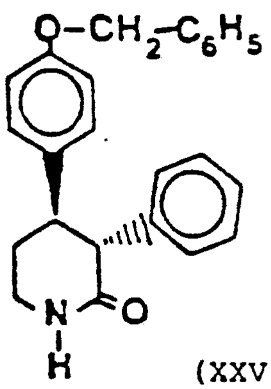

(XXV)

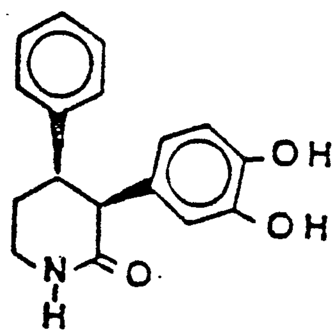

(XXVI)

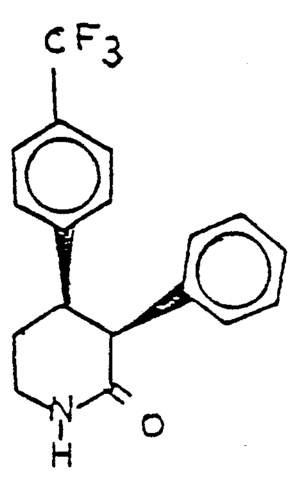

(XXVII)

7. Arzneimittel nach Anspruch 1, dadurch gekennzeichnet, dass sie ein Produkt der Formel A oder B zur Behandlung akuter und chronischer Niereninsuffizienz enthalten.

8. Psychostimulierende und antidepressive Arzneimittel nach Anspruch 1, dadurch gekennzeichnet, dass sie ein Produkt der Formel C enthalten.

Patentanspruch(üche) für den Vertragsstaat : AT

1. Verfahren zur Herstellung neuer Arzneimittel, dadurch gekennzeichnet, daß man 2,3-oder 3,4-Diphenyl-$\gamma$-nitril-esterderivate der Formeln A oder B

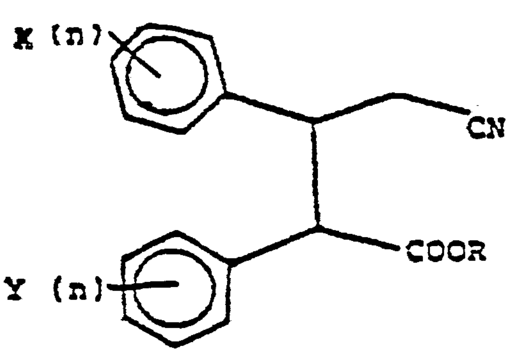

(A)

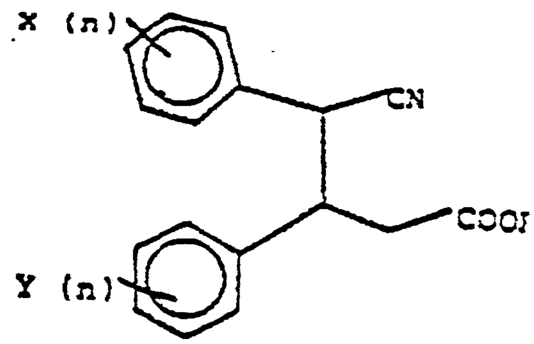

(B)

worin R für ein Wasserstoffatom, ein pharmazeutisch unbedenkliches Alkali-oder Erdalkalimetall oder eine $C_1$-$C_4$-Alkylgruppe sowie X und Y, die gleich oder verschieden sein können, für ein Wasserstoff- oder Halogenatom oder eine $C_1$-$C_4$-Alkyl-, $C_1$-$C_4$-Alkoxy-, Phenyl-, Phenyl-$C_1$-$C_4$-alkyl-oder Trifluormethylgruppe stehen, wobei n 1 bis 3 beträgt, falls X und/oder Y von Wasserstoff verschieden sind, oder Ringschlussprodukte der 2,3-Diphenylderivate, der Formel C

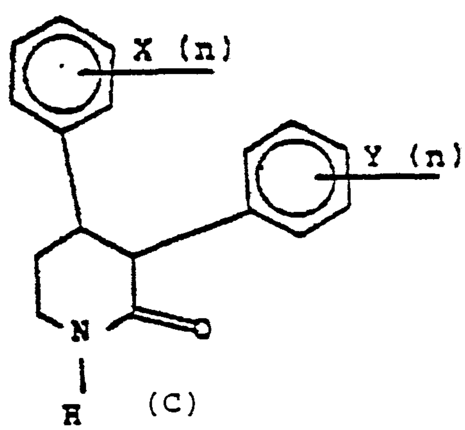

(C)

worin R für ein Wasserstoffatom, ein pharmazeutisch unbedenkliches Alkali-oder Erdalkalimetall oder eine $C_1$-$C_4$-Alkylgruppe sowie X und Y, die gleich oder verschieden sein können, für ein Wasserstoff- oder Halogenatom oder eine Trifluormethyl-, $C_1$-$C_4$-Alkyl-, Phenyl-, Phenyl-$C_1$-$C_4$-alkyl-, $C_1$-$C_4$-Alkoxy-, Hydroxyl-, Acyloxy-, Phenyloxy-, Benzyloxy-, $C_1$-$C_4$-Alkylsulfonyl-oder Di-$C_1$-$C_4$-alkylaminogruppe stehen, wobei n 1 bis 3 beträgt, falls X und/oder Y von Wasserstoff verschieden sind in eine geeignete Darreichungsform überführt.

2. Verfahren nach Anspruch 1, dadurch gekennzeichnet, dass die durch die Formeln A und B dargestellten Produkte die erythro-oder threo-bzw. im Fall der Piperidinone (Formel C) die cis-oder trans-Konfiguration besitzen.

3. Ringschlussverfahren zur Herstellung der 2,3-Diphenyl-γ-nitril-esterderivate der Formel C gemäß Anspruch 1 unter Festlegung der Stereochemie der entstehenden Piperidinone, dadurch gekennzeichnet, dass man, wenn ein reines cis-Derivat erwünscht ist, von einem reinen erythro-Produkt ausgeht und dieses bei einem Druck zwischen 10 und 80 bar und einer Temperatur zwischen 30 und 100°C hydriert bzw., wenn ein reines trans-Derivat erwünscht ist, entweder von einem reinen threo-Derivat oder einem erythro-Derivat ausgeht, das bei einem Druck zwischen 80 und 180 bar und einer Temperatur zwischen 80 und 180°C hydriert wird.

4. Verfahren zur Herstellung von neuen 2,3-Diphenyl-γ-nitril-esterderivaten der nachfolgenden Formeln I bis VIII:

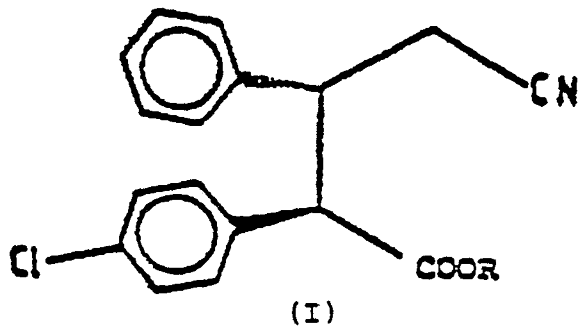

(I)

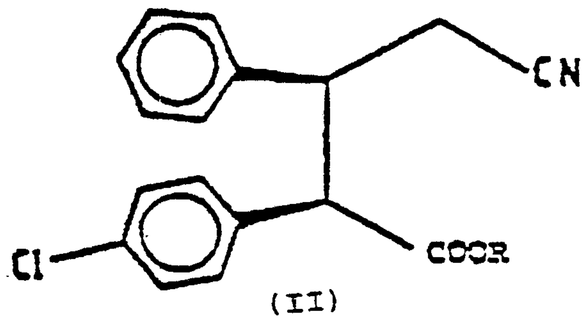

(II)

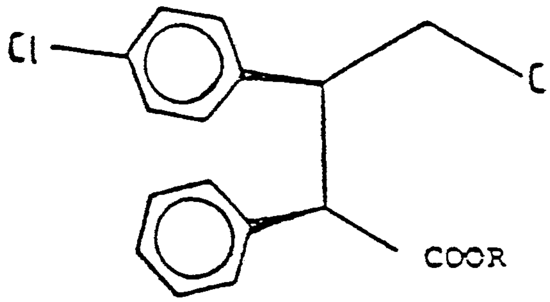

(III)

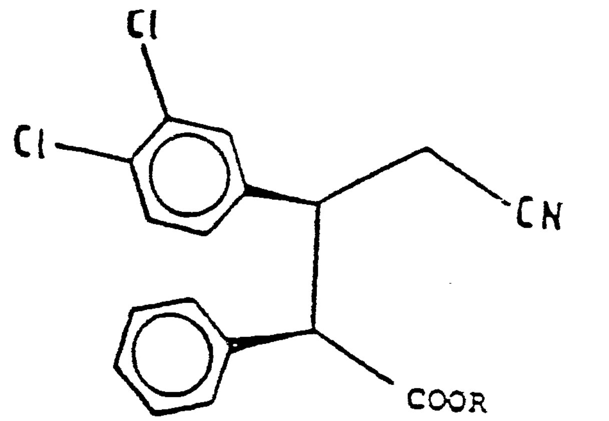

(IV)

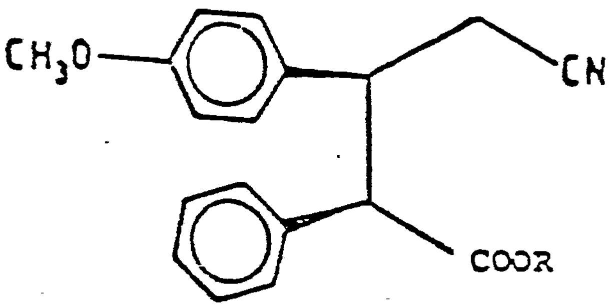

(V)

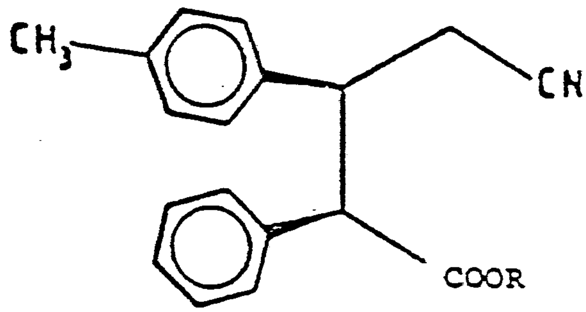

(VI)

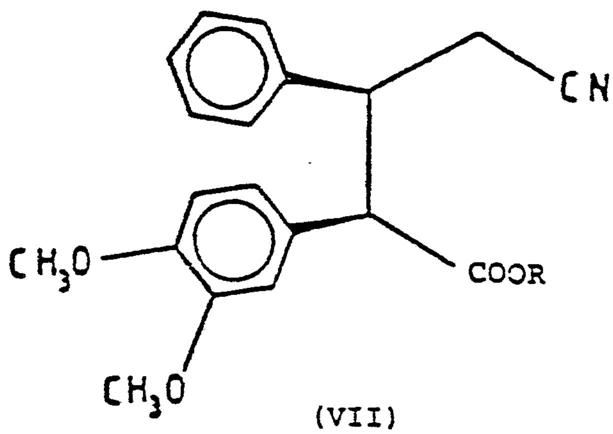

(VII)

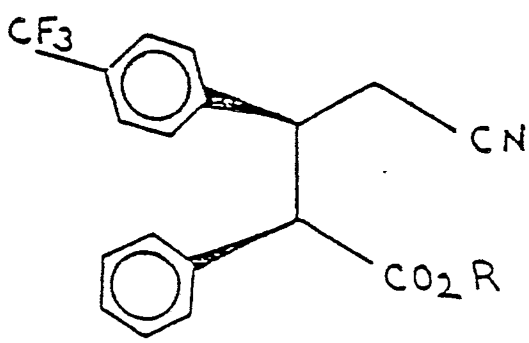

(VIII)

worin R für ein Wasserstoffatom, ein pharmazeutisch unbedenkliches Alkali-oder Erdalkalimetall oder eine $C_1$-$C_4$-Alkylgruppe steht, dadurch gekennzeichnet, daß man entsprechend substituierte Zimtsäurenitrile in Gegenwart von Natriumamid mit entsprechend substituierten Phenylessigsäureestern umsetzt.

5. Verfahren zur Herstellung von neuen 3,4-Diphenyl-γ-nitril-esterderivaten der Formeln IX und X

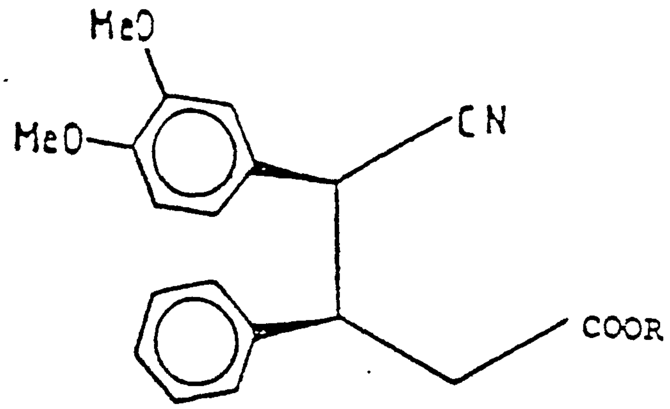

(IX)

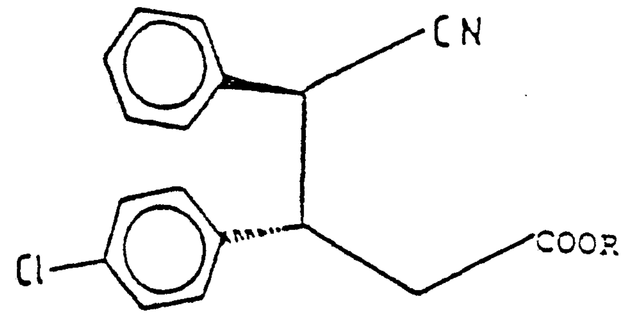

(X)

worin R für ein Wasserstoffatom, ein pharmazeutisch unbedenkliches Alkali-oder Erdalkalimetall oder eine $C_1$-$C_4$-Alkylgruppe steht, dadurch gekennzeichnet, daß man entsprechend substituierte Zimtsäureester in Gegenwart von Natriumalkoholat mit entsprechend substituierten Benzylcyamiden umsetzt.

6. Verfahren gemäß Anspruch 3, dadurch gekennzeichnet, daß man 3,4-Diphenyl-piperidinone der nachfolgenden Formeln XI bis XXVII:

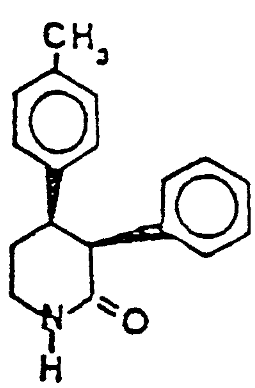

(XI)

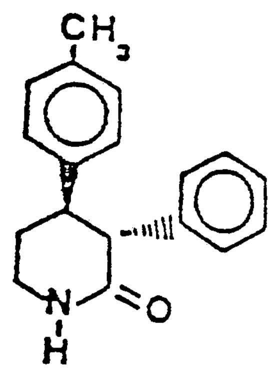

(XII)

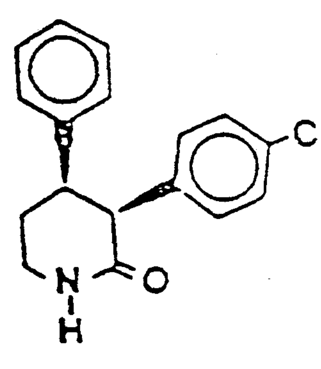

(XIII)

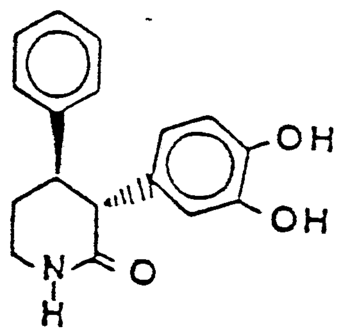

(XIV)

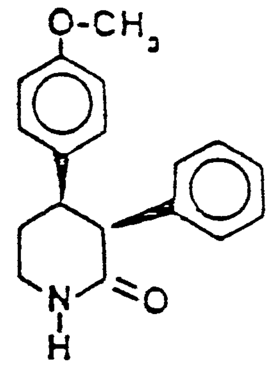

(XV)

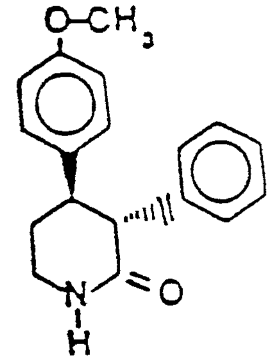

(XVI)

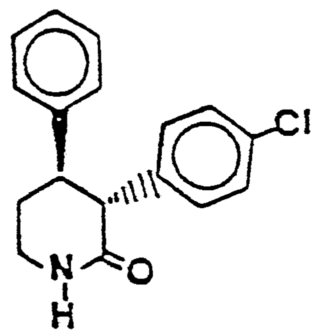

(XVII)

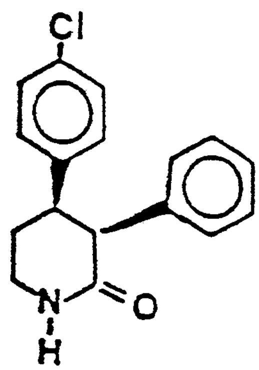

(XVIII)

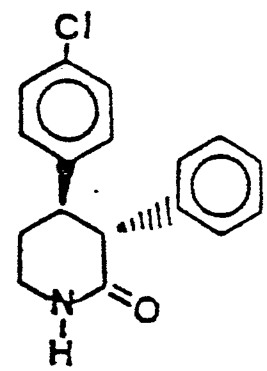

(XIX)

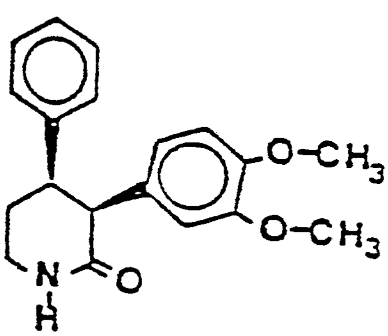

(XX)

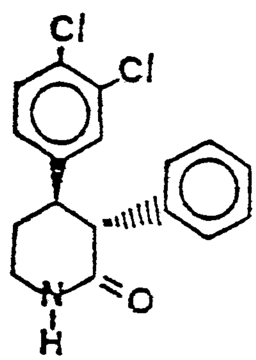

(XXI)

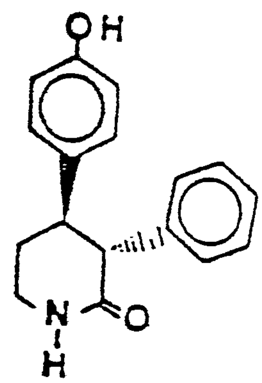

(XXII)

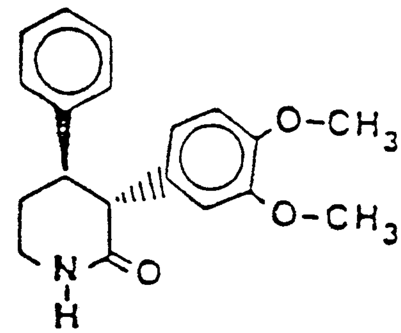

(XXIII)

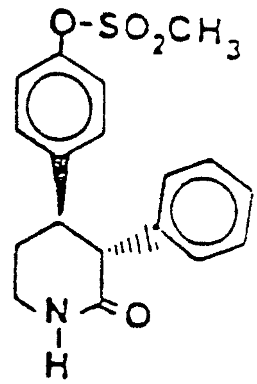

(XXIV)

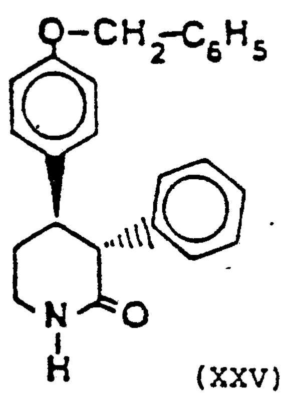

(XXV)

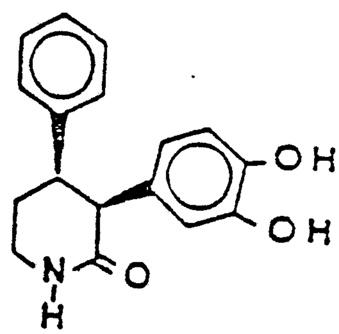

(XXVI)

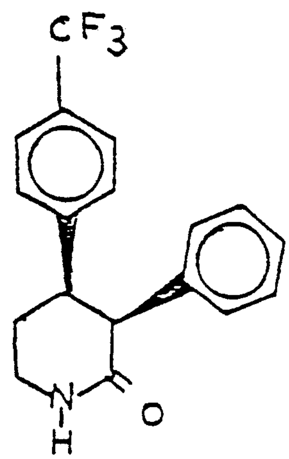

(XXVII)

herstellt.